# EUROPEAN PATENT APPLICATION

(11) **EP 4 793 288 A1**
(43) Date of publication of application: **19.08.2026**
(21) Application number: 24877590.0
(22) Date of filing: 14.10.2024
(51) Int. Cl.: C07K 16/28, A61P 35/00, G01N 33/574, A61K 39/00

(54) **ANTIBODY SPECIFICALLY BINDING TO HLA-G, AND USE THEREOF**

(30) Priority: 12.10.2023 KR 20230136277
(71) Applicant: Imbiologics Corp., Suwon-si Gyeonggi-do 16229 (KR)
(72) Inventor: HA, Gyong Sik, Seoul 07287 (KR); LEE, Chung Min, Gwacheon-si, Gyeonggi-do 13833 (KR); KANG, Hyeonju, Yongin-si, Gyeonggi-do 16949 (KR); LEE, Inyoung, Suwon-si, Gyeonggi-do 16423 (KR); JU, Man Seok, Seoul 08606 (KR)
(74) Representative: Rückerl, Florian
(86) International application number: PCT/KR2024/015542
(87) International publication number: WO 2025/080055

(57) **Abstract**

The present invention relates to an antibody specifically binding to human leukocyte antigen G (HLA-G) or an antigen-binding fragment thereof, and use thereof. The antibody specifically binding to HLA-G or an antigen-binding fragment thereof can exhibit excellent anticancer effects such as that of inhibiting tumor growth by inhibiting the interaction between HLA-G and ILT-2 while specifically binding to HLA-G.

## Description

### [Technical Field]

The present invention relates to an antibody or an antigen-binding fragment thereof that specifically binds to human leukocyte antigen G (HLA-G), and to uses thereof.

### [Background Art]

Although the origin of cancer is not fully understood, descriptions of cancer found in papyrus documents dating back to approximately 1600 BC, which were discovered in the late 19th century, suggest that cancer has long been a disease threatening humanity. Since the establishment of the American Cancer Society in 1913, extensive efforts have been made to overcome cancer; however, despite more than a century of research, cancer has yet to be conquered.

Efforts to overcome cancer are reflected in the development of anticancer agents. Beginning with the development of first-generation cytotoxic anticancer agents in the 1960s and 1970s, the field transitioned to second-generation targeted anticancer agents in the early 2000s. Subsequently, on March 28, 2011, the approval by the U.S. Food and Drug Administration of the use of the immune checkpoint CTLA-4 inhibitory antibody ipilimumab for the treatment of late-stage melanoma resulted in a paradigm shift toward third-generation immuno-oncology agents.

Thereafter, in September and December 2014, the U.S. Food and Drug Administration approved the use of the PD-1 inhibitory antibodies pembrolizumab and nivolumab for the treatment of melanoma. To date, a total of eight immune checkpoint inhibitory antibodies, including the aforementioned three agents, as well as the PD-1 inhibitors cemiplimab and dostarlimab, and the PD-L1 inhibitors atezolizumab, durvalumab, and avelumab, have been approved by the U.S. Food and Drug Administration for the treatment of solid tumors, and third-generation immuno-oncology agents are leading the anticancer drug market.

However, responsiveness to conventional immuno-oncology agents varies significantly depending on cancer type and individual patients, and in certain cancers, such as non-small cell lung cancer and ovarian cancer, the proportion of patients exhibiting resistance to conventional immune checkpoint inhibitory agents, including PD-1 and PD-L1 inhibitors, approaches 80% (Front. Immunol. 2020, 11:577869). In addition, the proportion of patients exhibiting acquired resistance, in which responsiveness is observed during initial treatment with the aforementioned agents but not observed upon subsequent treatment, is also very high. The causes of intrinsic resistance and acquired resistance are diverse, including mutations in downstream signaling molecules and expression of alternative immune checkpoint molecules, and researchers have reported that additional causes not yet identified may also exist (Front. Cell Dev. Biol. 2020, 8:672).

As described above, the proportion of patients exhibiting resistance to conventional immuno-oncology agents is very high, and the mechanisms of resistance are also highly complex. Accordingly, there is an unmet medical need for the identification of new immune checkpoints that are different from the target molecules of conventional immuno-oncology agents and for the development of inhibitory agents targeting the same.

Human leukocyte antigen G (HLA-G) is known to be expressed in the placenta of pregnant women and to function as an immune checkpoint that protects the fetus from the maternal immune system (Front. Immunol. 2020, 11:1685). Recently, as reports on the association between HLA-G and cancer have increased, HLA-G has attracted attention as an anticancer target molecule. HLA-G is a novel immune checkpoint target molecule that is different from CTLA-4, PD-1, and PD-L1, which are target molecules of conventional immuno-oncology agents. In particular, HLA-G is overexpressed in cancers in which conventional immuno-oncology agents exhibit low responsiveness, including non-small cell lung cancer, colorectal cancer, gastric cancer, ovarian cancer, and pancreatic cancer, and it has been reported that the expression of HLA-G has a negative effect on the prognosis of patients with the aforementioned cancers (Trends Immunol. 2008, 29(3):125; J. Int. Med. Res. 2020, 48(11):0300060520970758; Oncol. Lett. 2022, 24(1):226; Ann. Sug. Oncol. 2007, 14(10):2721; Hum. Immunol. 2018, 79(6):463; Cancer Sci. 2020, 111(8):3057).

The association between the expression of HLA-G and patient prognosis is related to the role of HLA-G in cancer. HLA-G overexpressed in cancer binds to molecules such as ILT-2 expressed on immune cells within the tumor microenvironment, inhibits the antibody-producing ability of B cells, inhibits the ability of NK and T cells to kill cancer cells, and promotes the differentiation of Treg cells to increase the secretion of anti-inflammatory cytokines, thereby impairing the immune system within the tumor microenvironment. Accordingly, HLA-G may be a highly attractive anticancer target molecule (Immunol. 2020, 11:1685).

Furthermore, while PD-1 and PD-L1 are tumor-associated antigens (TAAs) that are also expressed in normal tissues, HLA-G is a tumor-specific antigen (TSA) that is not expressed in normal tissues except for the placenta of pregnant women and is overexpressed only in cancer. Accordingly, concerns regarding targeting of normal tissues by HLA-G-specific agents are significantly reduced, and HLA-G may have higher value as an anticancer target molecule.

### [Technical Problem]

The present inventors have conducted research to develop a novel anticancer agent. As a result, an antibody or an antigen-binding fragment thereof that specifically binds to HLA-G and inhibits the interaction between HLA-G and ILT-2 was prepared, and it was confirmed that the antibody or antigen-binding fragment thereof has high binding affinity to HLA-G protein and exhibits excellent anticancer effects, thereby completing the present invention.

### [Technical Solution]

Each description and embodiment disclosed in the present invention may be applied to other descriptions and embodiments. That is, all combinations of the various elements disclosed in the present invention fall within the scope of the present invention. In addition, the scope of the present invention is not limited by the specific descriptions set forth below.

Further, terms not specifically defined in the present specification should be understood as having meanings commonly used in the art to which the present invention pertains. Unless otherwise clearly indicated by the context, a singular expression includes a plural expression, and a plural expression includes a singular expression.

One aspect of the present invention provides an antibody or an antigen-binding fragment thereof that specifically binds to human leukocyte antigen G (HLA-G).

As used herein, the term "human leukocyte antigen G (HLA-G)" refers to a protein encoded by the *HLA-G* gene, and is also referred to as histocompatibility antigen, class I, G. The amino acid sequence of HLA-G and the nucleotide sequence encoding HLA-G are known in publicly available databases (NCBI Reference Sequence: NP_002118, NP_001350496, NM_002127, NM_001363567, NM_001384280, NM_001384290, etc.).

As used herein, the term "anti-HLA-G antibody" refers to an antibody or an antigen-binding fragment thereof that specifically binds to an HLA-G protein to neutralize or inhibit the activity of the HLA-G protein, or that specifically binds to HLA-G to inhibit the interaction between HLA-G and an ILT-2 receptor. Specifically, the anti-HLA-G antibody may be a human antibody that targets a human HLA-G protein and also has reactivity to HLA-G proteins derived from mouse or rat.

As used herein, the term "antibody" refers to an immunoglobulin molecule having immunological reactivity to a specific antigen or a protein molecule that functions as a receptor recognizing an antigen specifically. Accordingly, the term "antibody" is used in a broad sense in the present invention and is construed to include polyclonal antibodies, monoclonal antibodies, whole antibodies (antibodies composed of at least two heavy chains and two light chains interconnected by disulfide bonds), and antibody fragments. The whole antibodies include IgA, IgD, IgE, IgM, and IgG. In addition, IgG may include subtypes IgG1, IgG2, IgG3, and IgG4. As used herein, the term "antibody fragment" refers to an "antigen-binding fragment" or an "antibody analog" that retains at least a portion of the binding specificity of a parent antibody and includes at least a part of the antigen-binding region of the parent antibody (for example, one or more CDRs) or a variable region. The antibody fragment may be, for example, a Fab, Fab', F(ab')₂, Fv fragment, scFv, unibody, diabody, linear antibody, nanobody, domain antibody, or a multispecific antibody formed from antibody fragments.

As used herein, the term "heavy chain" refers to a full-length heavy chain including a heavy chain variable region and a heavy chain constant region, and fragments thereof. The heavy chain includes gamma (γ), mu (µ), alpha (α), delta (δ), and epsilon (ε) types.

As used herein, the term "light chain" refers to a full-length light chain including a light chain variable region and a light chain constant region, and fragments thereof. The light chain includes kappa (κ) and lambda (λ) types.

As used herein, the term "complementarity determining region (CDR)" refers to an amino acid sequence of a hypervariable region forming an antigen-binding site, which is a part of a variable region of an antibody generated by B cells and T cells. The amino acid sequences of the heavy chain and the light chain each include three non-contiguously arranged CDRs, namely, heavy chain CDR_{H1}, CDR_{H2}, and CDR_{H3}, and light chain CDR_{L1}, CDR_{L2}, and CDR_{L3}. The CDR is a region involved in antigen recognition and provides major contact residues in binding of an antibody to an antigen or epitope, thereby playing a critical role in the diversity of antigen specificity.

The antibody of the present invention may be a whole antibody or an antibody fragment having antigen-binding ability, and the heavy chain may be any one of gamma (γ), mu (µ), alpha (α), delta (δ), or epsilon (ε) types, and the light chain may be kappa (κ) or lambda (λ) type.

In one embodiment of the present invention, the antibody or the antigen-binding fragment thereof may comprise a heavy chain CDR1 represented by an amino acid sequence of SEQ ID NO: 1, 17, 33, 49, 64, 79, 94, 109, 125, 140, 156, 172, or 188; a heavy chain CDR2 represented by an amino acid sequence of SEQ ID NO: 2, 18, 34, 50, 65, 80, 95, 110, 126, 141, 157, 173, or 189; and a heavy chain CDR3 represented by an amino acid sequence of SEQ ID NO: 3, 19, 35, 51, 66, 81, 96, 111, 127, 142, 158, 174, or 190.

Further, the antibody or the antigen-binding fragment thereof may comprise a light chain CDR1 represented by an amino acid sequence of SEQ ID NO: 4, 20, 36, 52, 67, 82, 97, 112, 128, 143, 159, 175, or 191; a light chain CDR2 represented by an amino acid sequence of SEQ ID NO: 5, 21, 37, 53, 68, 83, 98, 113, 129, 144, 160, 176, or 192; and a light chain CDR3 represented by an amino acid sequence of SEQ ID NO: 6, 22, 38, 54, 69, 84, 99, 114, 130, 145, 161, 177, or 193.

Further, the antibody or the antigen-binding fragment thereof may comprise a heavy chain variable region represented by an amino acid sequence of SEQ ID NO: 15, 31, 47, 123, 154, 170, 186, or 202.

Further, the antibody or the antigen-binding fragment thereof may comprise a light chain variable region represented by an amino acid sequence of SEQ ID NO: 16, 32, 48, 63, 78, 93, 108, 124, 139, 155, 171, 187, or 203.

The sequences disclosed in the present invention include sequences that are substantially identical to the sequences set forth in the Sequence Listing. As used herein, "substantial identity" means that, when two sequences are aligned to correspond as closely as possible and analyzed using an algorithm commonly used in the art, the sequences exhibit sequence homology of 80%, 90%, or 95% or more.

Further, the antibody of the present invention or the antigen-binding fragment thereof may include, within a range capable of specifically recognizing and binding to an HLA-G protein, not only the sequences of the antibody or antigen-binding fragment described herein and sequences that are substantially identical thereto, but also biological equivalents thereof. For example, it may include additional variations in the sequence for improving antibody binding affinity and/or biological properties, and may include additional variations within a range that does not alter the overall activity of the molecule.

Accordingly, sequences having high homology with the sequences represented by SEQ ID NOs: 1 to 223 of the present invention, for example, sequences having a homology of 70% or more, specifically 80% or more, and more specifically 90% or more, should be construed as being included within the scope of the present invention.

In one embodiment of the present invention, the antibody of the present invention or the antigen-binding fragment thereof may be conjugated with a drug to form an antibody-drug conjugate (ADC). In the antibody-drug conjugate, the drug to which the antibody of the present invention or the antigen-binding fragment thereof is conjugated may also be referred to as a "payload". As long as the antibody of the present invention or the antigen-binding fragment thereof is used, the drug used in the antibody-drug conjugate may be, for example, one that has already been approved for marketing or is currently under development, but is not limited thereto.

In one embodiment of the present invention, the antibody of the present invention or the antigen-binding fragment thereof may be included in a fusion protein or may constitute a fusion protein. In another embodiment of the present invention, a fusion protein may comprise the antibody of the present invention or the antigen-binding fragment thereof.

As used herein, the term "fusion protein" refers to a protein in which parts or all of two or more different proteins are combined. The fusion protein includes amino acid sequences of the proteins constituting the same, and may further include, but not limited to, an amino acid sequence such as a signal peptide that allows a protein produced in a cell to be secreted outside the cell.

Specifically, the fusion protein may comprise a biologically active molecule, a linker, an IgG Fc region, an IgM Fc region, and a J chain, and more specifically may be represented by the following formula:

[(X)ₐ-(Y)_{b}-(IgG Fc region)_{c}-(IgM Fc region)]ₘ-(Z)ₙ

wherein
X is a biologically active molecule,
Y is a linker,
IgG Fc region is a monomer,
IgM Fc region is a monomer,
Z is a J chain,
a is 1 or 2, b is 1 or 2, c is 0 or 1, m is 5 or 6, and n is 0 or 1.

As used herein, the term "IgG Fc region" refers to an Fc region (fragment crystallizable region) of immunoglobulin G (IgG), and means a portion of IgG excluding an Fab region (fragment antigen-binding region). The IgG Fc region may be directly linked to a linker and an IgM Fc region in the fusion protein. In addition, the IgG Fc region may be indirectly linked to a biologically active molecule via a linker in the fusion protein, and in this case, the IgG Fc region may serve as a connector, scaffold, or carrier that allows the inclusion of one or two or more biologically active molecules. As used herein, the "IgG Fc region" may be used interchangeably with "IgG Fc fragment".

Specifically, the IgG Fc region monomer may be composed of two heavy chains. Each heavy chain may include one or more selected from the group consisting of a CH2 domain and a CH3 domain. For example, each heavy chain may include or consist of a CH2 domain and a CH3 domain.

In the present invention, the IgG Fc region may be derived from IgG1, IgG2, IgG3, or IgG4.

In addition, the IgG Fc region may be derived from a wild-type IgG or a variant IgG. In this case, the variant IgG may have mutations that reduce antibody-dependent cell-mediated cytotoxicity (ADCC) and/or mutations that reduce complement-dependent cytotoxicity (CDC).

As used herein, the term "IgM Fc region" refers to an Fc region (fragment crystallizable region) of immunoglobulin M (IgM), and means a portion of IgM excluding an Fab region (fragment antigen-binding region). The IgM Fc region may be directly linked to an IgG Fc region in the fusion protein, and may also be directly linked to a linker in the fusion protein when the IgG Fc region is not present. In addition, the IgM Fc region may be indirectly linked to a biologically active molecule via an IgG Fc region and a linker in the fusion protein, or may be indirectly linked to a biologically active molecule via a linker alone in the fusion protein. In this case, the IgM Fc region may serve as a connector, scaffold, or carrier that allows the inclusion of one or two or more biologically active molecules. As used herein, the "IgM Fc region" may be used interchangeably with "IgM Fc fragment."

In the present invention, the IgM Fc region monomer may form a pentamer or a hexamer, and each monomer may be linked via a disulfide bond.

Specifically, the IgM Fc region monomer may be composed of two heavy chains. Each heavy chain may include one or more selected from the group consisting of a Cµ2 domain, a Cµ3 domain, and a Cµ4 domain.

As used herein, the term "J chain (joining chain)" refers to a polypeptide that is a component of an IgM or IgA antibody and that forms a multimer by binding IgM or IgA monomers. The J chain may be present between IgM Fc region monomers in the fusion protein and may function to link them. Specifically, the J chain may be included when the IgM Fc region monomers form a pentamer, and may not be included when the IgM Fc region monomers form a hexamer. More specifically, when the IgM Fc region monomers form a pentamer, the J chain may be linked to a first IgM Fc region monomer and a fifth IgM Fc region monomer via disulfide bonds.

Specifically, the J chain may be derived from a wild-type J chain or a variant J chain.

In the present invention, the J chain may further have a protein molecule bound thereto. In this case, the protein molecule may exhibit a synergistic effect with respect to the intended effect of the fusion protein of the present invention. Specifically, it may be one or more selected from the group consisting of an antibody, an antigen-binding fragment of an antibody, an antibody-drug conjugate, an antibody-like molecule, an antigen-binding fragment of an antibody-like molecule, a soluble protein, a membrane-bound protein, a ligand, a receptor, a virus-like particle, a protein toxin, and an enzyme, but is not limited thereto. More specifically, it may be one or more selected from the group consisting of a co-stimulatory receptor, a cytokine, and a cell engager. The cell engager may be an antibody, specifically an antibody that binds to a cancer cell- or immune cell-specific marker protein, for example, a T-cell engager. In addition, the immune cells may include neutrophils, eosinophils, basophils, mast cells, monocytes, macrophages, dendritic cells, natural killer cells (NK cells), and lymphocytes such as B cells and T cells.

As used herein, the term "biologically active molecule" refers to a substance capable of exhibiting a specific action or effect in a living organism. For example, it may perform functions such as, by activating or inactivating a particular signaling pathway, promoting or inhibiting the expression of a specific gene, enhancing or inhibiting the function of a specific protein, or inducing or inhibiting cell death. Preferably, it may exhibit immune-regulating and/or anticancer activity.

Specifically, the biologically active molecule may be or comprise the antibody of the present invention or the antigen-binding fragment thereof.

In the present invention, the biologically active molecule may be indirectly linked to an IgG Fc region via a linker in the fusion protein, or may be indirectly linked to an IgM Fc region via a linker in the fusion protein. In this context, it may function as a functional group or an active moiety capable of providing the intended effect of the fusion protein.

Specifically, it may be one or more selected from the group consisting of an antibody, an antigen-binding fragment of an antibody, an antibody-drug conjugate, an antibody-like molecule, an antigen-binding fragment of an antibody-like molecule, a soluble protein, a membrane-bound protein, a ligand, a receptor, a virus-like particle, a protein toxin, a chemokine, a cytokine, and an enzyme, but is not limited thereto. The receptor may be a cytokine receptor and/or an immune checkpoint receptor, but is not limited thereto. For example, the fusion protein may comprise two or more types of biologically active molecules depending on the desired purpose, target, or effect.

As a specific example, the antigen-binding fragment of the antibody may be one or more selected from the group consisting of Fab, F(ab')₂, F(ab)₂, Fab', Fab₂, Fab₃, Fv, scFv, bis-scFv, minibody, triabody, diabody, tandem diabody (TandAb), nanobody and tetrabody, but is not limited thereto. In addition, the antigen-binding fragment of the antibody may bind to HLA-G. HLA-G is a type of class I HLA, and is expressed in the placenta of pregnant women, where it is involved in fetal immune tolerance. HLA-G binds to inhibitory receptors of natural killer (NK) cells, such as ILT2, ILT4, and KIR2DL4, thereby inhibiting NK cells and promoting the differentiation of regulatory T cells (Treg). In addition, when cancer cells overexpress HLA-G on their surface, the activity of cytotoxic T cells, helper T cells, and NK cells involved in cytotoxicity is suppressed, thereby reducing the efficacy of immunotherapy for cancer treatment. Accordingly, an antigen-binding fragment that selectively binds to HLA-G and inhibits the binding of HLA-G to inhibitory receptors may be used as an anticancer therapeutic agent. For example, the antigen-binding fragment of the antibody may be a Fab or scFv.

As another specific example, the receptor may be a human leukocyte antigen (HLA), but is not limited thereto. As used herein, the term "HLA" refers to proteins encoded by genes of the major histocompatibility complex (MHC) in humans, which are important immunological molecules that primarily exist in a membrane-bound form and are involved in the regulation of the immune system. HLA presents peptide fragments derived from foreign or self proteins from within a cell to the cell surface, and enables recognition and binding by T cell receptor (TCR) of cytotoxic T cells, thereby activating the cytotoxic T cells. HLA is broadly divided into two types: class I HLA is present on the surface of all cells, whereas class II HLA is present only on specific antigen-presenting cells (APCs), such as NK cells, macrophages, and dendritic cells. HLA genes exhibit a high degree of polymorphism and express six distinct HLA protein α chains (two HLA-A, two HLA-B, and two HLA-C). Class I HLA can selectively activate cytotoxic T cells expressing TCR when a specific peptide fragment is loaded thereon, and thus recombinant class I HLA in which the transmembrane domain is removed has been used for the treatment of cancer or infectious diseases. In addition, unlike class II HLA, most class I HLA molecules are unstable in the absence of a peptide loaded in the groove. Accordingly, in the production of recombinant class I HLA, a target synthetic peptide is loaded, or a recombinant HLA protein is produced in a form fused to the α chain. When expressed using microorganisms, there is a concern that proper refolding may not occur, and thus expression using animal cells is generally employed. In the art, HLA and MHC are used interchangeably to refer to the same meaning, and in humans, the term HLA is used instead of MHC. In the present specification, "HLA" and "MHC" may be used interchangeably.

The HLA may be class I HLA such as HLA-A, HLA-B, HLA-C, HLA-E, HLA-F, and HLA-G, or class II HLA such as HLA-DP, HLA-DM, HLA-DOA, HLA-DOB, HLA-DQ, and HLA-DR. The class I HLA consists of a β2m domain, an α1 domain, an α2 domain, and an α3 domain, and the class II HLA consists of an α1 domain, an α2 domain, a β1 domain, and a β2 domain. The HLA of the present invention may include a β2m domain, an α1 domain, an α2 domain, or an α3 domain, and may include an α1 domain, an α2 domain, a β1 domain, and a β2 domain.

In addition, the HLA may include a substance that specifically binds to an activation site of a target T cell. The substance may selectively activate a target T cell expressing a TCR and/or increase the stability of the HLA. As used herein, the term "target T cell" may refer to a T cell to which the fusion protein of the present invention binds, and may be a cytotoxic T cell (killer T cell; Tc) or a helper T cell (Th). The cytotoxic T cell is a cell capable of killing cancer cells, virus-infected cells, or damaged cells, and may be, for example, a CD8⁺ T cell. CD8, a protein receptor of CD8⁺ T cells, is located on the cell surface and binds to class I HLA. The helper T cell does not directly kill cells but functions to activate other immune cells, for example, by promoting antibody production by B cells and activation of cytotoxic T cells, and may be, for example, a CD4⁺ T cell. CD4, a protein receptor of CD4⁺ T cells, is located on the cell surface and binds to class II HLA.

A specific example of a substance that specifically binds to an activation site of the target T cell may be a peptide, but is not limited thereto. The peptide may be derived from a virus or an animal cell. The virus may be cytomegalovirus (CMV), but is not limited thereto. CMV is one of the most immunogenic antigens to the human immune system and induces a very strong CD8⁺ T cell response upon infection in humans. The CD8⁺ T cell immune response is primarily activated by CMV proteins pp65 and IE-1, and the frequency of CMV-specific T cells against individual peptides is known to be very high, reaching up to about 1% to 2% of the total CD8⁺ T cell repertoire.

As another specific example, the ligand may be erythropoietin (EPO) or an EPO analog, but is not limited thereto. As used herein, the term "EPO" refers to a hormone protein essential for erythropoiesis, which acts on hematopoietic stem cells in the bone marrow to promote differentiation and proliferation of red blood cells, thereby treating anemia. EPO also exerts neuroprotective effects under hypoxic conditions such as stroke and is involved in wound healing processes. In addition, EPO has been reported to be involved in hippocampal responses, synaptic connections, and neuronal networks in the brain, thereby contributing to memory enhancement and having therapeutic effects on depression. The "EPO analog" refers to a recombinant protein having a structure different from that of EPO but performing the same function, and having two additional glycan chains in addition to the three glycan chains of human EPO, thereby exhibiting a plasma half-life increased by three-fold or more compared to human EPO. The EPO analog may be a ligand, an antibody, or an antibody fragment capable of binding to and activating an EPO receptor, and a specific example thereof may be novel erythropoiesis stimulating protein (NESP), but is not limited thereto.

As used herein, the term "linker" refers to amino acids present between domains of a protein. The linker may be present between a biologically active molecule and an IgG Fc region in the fusion protein and may directly link the biologically active molecule and the IgG Fc region. In addition, when the IgG Fc region is not present in the fusion protein, the linker may be present between the biologically active molecule and an IgM Fc region and may directly link the biologically active molecule and the IgM Fc region.

As a specific example, the linker may be one or more selected from the group consisting of an IgD hinge region, an IgA hinge region, an IgG hinge region and a GS linker.

As used herein, the term "hinge region" refers to a region located in the central portion of a heavy chain of an antibody, which connects two domains of the heavy chain via disulfide bonds. The hinge region is located between the CH1 domain and the CH2 domain of the heavy chain and is connected thereto. Unlike immunoglobulins having a short hinge region that exhibit a Y-shaped structure, IgD specifically has a long hinge region, thereby providing sufficient flexibility such that the two target-binding sites can be spaced apart to form a T-shaped structure. This flexibility allows IgD to exhibit binding activity to multimeric foreign antigens while having little or no binding to self-antigens. As used herein, the "hinge region" may be used interchangeably with "hinge domain."

In the present invention, the hinge region may include a part or all of the hinge region, a part or all of the CH1 domain, a part or all of the CH2 domain, or a combination thereof.

For example, the hinge region may include part or all of an IgD hinge (hereinafter, "part or all" may be expressed as "part/all"). Specifically, the hinge region may include part/all of an IgD hinge and part/all of an IgD CH1 domain. Specifically, the hinge region may include part/all of an IgD hinge and part/all of an IgD CH2 domain. Specifically, the hinge region may include part/all of an IgD hinge, part/all of an IgD CH1 domain, and part/all of an IgD CH2 domain.

As another example, the hinge region may include part/all of an IgA hinge. Specifically, the hinge region may include part/all of an IgA hinge and part/all of an IgA CH1 domain. Specifically, the hinge region may include part/all of an IgA hinge and part/all of an IgA CH2 domain. Specifically, the hinge region may include part/all of an IgA hinge, part/all of an IgA CH1 domain, and part/all of an IgA CH2 domain.

As another example, the hinge region may include part/all of an IgG hinge. Specifically, the hinge region may include part/all of an IgG hinge and part/all of an IgG CH1 domain. Specifically, the hinge region may include part/all of an IgG hinge and part/all of an IgG CH2 domain. Specifically, the hinge region may include part/all of an IgG hinge, part/all of an IgG CH1 domain, and part/all of an IgG CH2 domain.

The "GS linker" may be one or more selected from the group consisting of (GS)_{d}, (SG)_{d}, (GGGS)_{d}, (GGGGS)_{d}, GCGS(GGGS)_{d}, and GCGGS(GGGGS)_{d}, wherein d is an integer of 2 to 6. Specifically, it may include 4 to less than 35 amino acids composed of glycine (Gly; G) and serine (Ser; S), and may include 4 to less than 35 amino acids composed of glycine, serine and cysteine (Cys; C) in order to enhance stability via disulfide bond, but is not limited thereto.

Another aspect of the present invention provides a nucleic acid encoding the antibody or the antigen-binding fragment thereof.

In the nucleic acid according to the present invention, unless otherwise specified, the relevant terms are to be understood as having the same meanings as those described above.

As used herein, the term "nucleic acid" encompasses DNA (including gDNA and cDNA) and RNA. The nucleotides constituting the basic structural units of a nucleic acid molecule include not only natural nucleotides but also nucleotide analogs in which the sugar or base moiety is modified (Scheit, Nucleotide Analogs, John Wiley, New York (1980); Uhlman and Peyman, Chemical Reviews, 90:543-584 (1990)).

The nucleic acid molecule encoding the antibody or the antigen-binding fragment thereof according to the present invention comprises a nucleotide sequence encoding the amino acid sequence constituting the antibody or the antigen-binding fragment thereof, and is not limited to any particular nucleotide sequence.

The nucleotide sequence includes a nucleotide sequence comprising codons that are functionally equivalent or that encode the same amino acid, in consideration of the degeneracy of codons (e.g., due to codon degeneracy, there are six codons for arginine or serine), or codons that encode biologically equivalent amino acids.

In view of the above-described variations having biologically equivalent activity, the nucleic acid molecule of the present invention encoding the antibody or the antigen-binding fragment thereof includes sequences having substantial identity thereto. As used herein, the term "substantial identity" means that, when two sequences are aligned to correspond to each other as much as possible and analyzed using an algorithm commonly used in the art, the sequences exhibit at least 80%, 90%, or 95% homology.

Another aspect of the present invention provides a vector comprising the nucleic acid and an isolated host cell transformed with the vector.

In the vector and the transformed host cell according to the present invention, unless otherwise specified, the relevant terms are to be understood as having the same meanings as those described above.

As used herein, the term "vector" refers to any entity capable of being introduced into a host cell and replicating a gene therein. Such vectors include plasmids, linear nucleic acids, cosmids, RNA vectors, and viral vectors. The viral vectors include, but are not limited to, retroviruses, adenoviruses, and adeno-associated viruses. The recombinant vector system of the present invention may be constructed by various methods known in the art. In addition, the vector of the present invention may be constructed as a vector for cloning or expression, and may be constructed using a prokaryotic cell or a eukaryotic cell as a host. Specifically, the vector may be a vector for expressing the antibody or the antigen-binding fragment thereof according to the present invention.

As used herein, the term "host cell" refers to a cell that comprises the vector and is capable of stably and continuously cloning and expressing the antibody or the antigen-binding fragment thereof according to the present invention. Examples thereof include prokaryotic host cells such as *Escherichia coli*; *Bacillus* species including *Bacillus subtilis* and *Bacillus thuringiensis*; *Streptomyces*, *Pseudomonas*, *Proteus mirabilis*, or *Staphylococcus*; eukaryotic host cells such as fungi including *Aspergillus* species, *Pichia pastoris*, *Saccharomyces cerevisiae*, *Schizosaccharomyces*, or *Neurospora crassa*; lower eukaryotic cells; higher eukaryotic cells such as insect-derived cells; plant cells; or mammalian cells such as COS-7 cells (monkey kidney cells), NSO cells, SP2/0 cells, Chinese hamster ovary (CHO) cells, WI-38 cells, baby hamster kidney (BHK) cells, MDCK cells, myeloma cell lines, HuT 78 cells, or 293 cells, but are not limited thereto. Any host cells commonly used in the art may be used without limitation.

Another aspect of the present invention provides a method for producing the antibody or the antigen-binding fragment thereof, comprising introducing the vector into a host cell.

In the method for producing the antibody or the antigen-binding fragment thereof according to the present invention, unless otherwise specified, the relevant terms are to be understood as having the same meanings as those described above.

Specifically, the method for producing the antibody or the antigen-binding fragment thereof according to the present invention may comprise:
(a) introducing the vector of the present invention into a host cell;
(b) culturing the host cell; and
(c) obtaining the antibody or the antigen-binding fragment thereof from the host cell.

The step (a) of introducing the vector of the present invention into a host cell may be a step of preparing a transformant comprising a vector for expressing the antibody or the antigen-binding fragment thereof, and the step of preparing the transformant may comprise transforming the host cell.

As used herein, the term "transformant" refers to an organism in which foreign DNA has been introduced into a cell, thereby resulting in an artificially induced genetic modification, such that the introduced DNA becomes capable of replication as a chromosomal component or by integration into the chromosome.

Transformation of the host cell described above may be performed using any transformation method, and may be readily carried out according to methods commonly used in the art. For example, it may include CaCl₂ transformation, the Hanahan method, which is a CaCl₂-based transformation method using dimethyl sulfoxide (DMSO) to enhance efficiency, electroporation, calcium phosphate precipitation, protoplast fusion, agitation using silicon carbide fibers, Agrobacterium-mediated transformation, PEG-mediated transformation, dextran sulfate-mediated transformation, lipofectamine-mediated transformation, and drying- or heat shock-mediated transformation, but is not limited thereto. Any transformation or transfection methods commonly used in the art may be used without limitation.

The step (b) of culturing the host cell of the present invention may be carried out in accordance with media and culture conditions known in the art. Such culture processes may be readily adjusted and employed by those skilled in the art depending on the selected strain. Depending on the growth mode of the cells, suspension culture or adherent culture may be employed; and depending on the culture method, batch, fed-batch, or continuous culture may be used.

In animal cell culture, the medium may include various carbon sources, nitrogen sources, and trace elements. Examples of carbon sources include carbohydrates such as glucose, sucrose, lactose, fructose, maltose, starch, or cellulose; lipids such as soybean oil, sunflower oil, castor oil, or coconut oil; fatty acids such as palmitic acid, stearic acid, or linoleic acid; alcohols such as glycerol or ethanol; or organic acids such as acetic acid, and these carbon sources may be used alone or in combination. Examples of nitrogen sources include organic nitrogen sources such as peptone, yeast extract, meat extract, malt extract, corn steep liquor, or soybean meal; or inorganic nitrogen sources such as urea, ammonium sulfate, ammonium chloride, ammonium phosphate, ammonium carbonate, or ammonium nitrate, and these nitrogen sources may be used alone or in combination. Examples of trace components include phosphorus sources such as potassium dihydrogen phosphate or dipotassium hydrogen phosphate, and metal salts such as magnesium sulfate or ferrous sulfate. In addition, amino acids, vitamins, or suitable precursors may be included.

In addition, in order to adjust the pH of the culture, compounds such as ammonium hydroxide, potassium hydroxide, ammonia, phosphoric acid, or sulfuric acid may be added in an appropriate manner to the culture during cultivation. Further, in order to suppress foam formation, an antifoaming agent such as a fatty acid polyglycol ester may be used during cultivation, and in order to maintain an aerobic state of the culture, oxygen or an oxygen-containing gas (e.g., air) may be supplied into the culture.

In addition, the culture may be carried out while maintaining the temperature of the culture at 20°C to 45°C, specifically 25°C to 40°C.

The step (c) of expressing the antibody or the antigen-binding fragment thereof in the host cell of the present invention may be a step of recovering, purifying, and concentrating the antibody or the antigen-binding fragment thereof from the culture obtained in step (b).

The antibody or the antigen-binding fragment thereof may be used without purification, or may be used after further recovering, purifying, and concentrating. Specifically, methods commonly used in the art (e.g., dialysis, salt precipitation, chromatography, etc.) may be employed, whereby recovery, purification, and concentration may be performed simultaneously. More specifically, the antibody or the antigen-binding fragment thereof may be recovered, purified, and concentrated using chromatography (e.g., ion-exchange chromatography, size-exclusion chromatography, or affinity chromatography), and the type and order of columns used therefor may be appropriately selected depending on the properties of the antibody or the antigen-binding fragment thereof and the culture method.

Another aspect of the present invention provides a pharmaceutical composition for preventing or treating cancer, comprising the antibody or the antigen-binding fragment thereof.

In the pharmaceutical composition according to the present invention, unless otherwise specified, the relevant terms are to be understood as having the same meanings as those described above.

As used herein, the term "treatment" refers to all actions in which cancer is ameliorated, reversed, or cured by administration of the composition according to the present invention. In addition, the term "prevention" refers to all actions in which the onset or recurrence of cancer is inhibited, delayed, or prevented by administration of the composition according to the present invention.

Specifically, the cancer may be one or more selected from the group consisting of lung cancer, non-small cell lung cancer, colorectal cancer, rectal cancer, perianal cancer, colon cancer, small intestine cancer, breast cancer, uterine cancer, ovarian cancer, fallopian tube carcinoma, endometrial carcinoma, cervical carcinoma, vaginal carcinoma, vulvar carcinoma, head cancer, neck cancer, thyroid cancer, parathyroid cancer, gastric cancer, liver cancer, pancreatic cancer, bone cancer, skin cancer, melanoma of the skin or intraocular melanoma, Hodgkin's disease, esophageal cancer, endocrine gland cancer, adrenal cancer, soft tissue sarcoma, urethral cancer, penile cancer, prostate cancer, bladder cancer, kidney cancer, ureteral cancer, renal cell carcinoma, renal pelvis carcinoma, central nervous system tumors, central nervous system lymphoma, spinal cord tumors, glioblastoma, brainstem glioma, and pituitary adenoma, but is not limited thereto.

In addition, the cancer may exhibit resistance or refractoriness to anticancer agents. As used herein, the term "resistance to anticancer agents" refers to a condition in which, upon treatment of a cancer patient with an anticancer agent, no therapeutic effect is observed from the beginning of treatment, or an initial therapeutic effect is observed but is subsequently reduced or lost during continued treatment. In addition, the term "refractoriness to anticancer agents" refers to a condition in which, upon treatment of a cancer patient with an anticancer agent, no therapeutic effect is observed from the beginning of treatment, or the response to treatment is not sustained over a prolonged period.

The pharmaceutical composition of the present invention comprising the antibody or the antigen-binding fragment thereof in an effective amount may be administered to a subject in need of prevention or treatment of cancer.

As used herein, the term "administration" refers to physically introducing the composition into a subject using any of a variety of methods and delivery systems known to those skilled in the art. The administration may be performed, for example, by oral administration, or by intravenous, intramuscular, subcutaneous, intraperitoneal, spinal, or other parenteral administration, such as by injection or infusion, but is not limited thereto. It may be performed, for example, as a single administration, multiple administrations, or over one or more extended periods.

As used herein, the term "subject" includes a human or any non-human animal, including a vertebrate such as a primate, dog, cow, horse, pig, or rodent, for example, a mouse, rat, or guinea pig. As used herein, the term "subject" is used interchangeably with "individual" and "patient".

In addition, the effective amount may be a "therapeutically effective amount" or a "prophylactically effective amount". As used herein, the term "therapeutically effective amount" refers to any amount of a drug or therapeutic agent that, when used alone or in combination with other therapeutic agents, is capable of reducing the severity of disease symptoms, increasing the frequency and duration of symptom-free periods, or preventing damage or disability resulting from the disease. The term "prophylactically effective amount" refers to any amount that inhibits the onset or recurrence of cancer in a subject. The level of the effective amount may be determined depending on factors such as the severity of the subject, age, sex, activity of the drug, sensitivity to the drug, time of administration, route of administration, rate of excretion, duration of treatment, concomitant drugs, and other factors well known in the medical field.

In addition, the pharmaceutical composition may vary depending on the age, sex, and body weight of the subject. Specifically, it may be administered at a dose of 0.1 to 100 mg/kg to a subject once or several times per day, or at intervals of several days to several months, depending on the symptoms of the subject. In addition, the dosage may be increased or decreased depending on the route of administration, severity of the disease, sex, body weight, age, and the like.

In addition, the pharmaceutical composition may further comprise suitable carriers, excipients, and diluents commonly used in the preparation of pharmaceutical compositions. Examples of carriers, excipients, and diluents that may be comprised in the composition include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

In addition, the pharmaceutical composition may be administered in combination with other therapeutic agents. In such cases, the pharmaceutical composition of the present invention and the other therapeutic agents may be administered simultaneously, sequentially, or separately. The other therapeutic agents may include, but are not limited to, compounds or proteins having preventive, therapeutic, or ameliorative effects on cancer.

In addition, the pharmaceutical composition may be formulated for administration with other therapeutic agents simultaneously, sequentially, or separately. For example, the antibody or the antigen-binding fragment thereof and the other therapeutic agents may be administered simultaneously as a single formulation, or may be administered simultaneously, sequentially, or separately as separate formulations. For simultaneous, sequential, or separate administration, the antibody or the antigen-binding fragment thereof comprised in the pharmaceutical composition of the present invention and the other therapeutic agents may be formulated in separate containers or together in a single container. In addition, the antibody or the antigen-binding fragment thereof comprised in the pharmaceutical composition of the present invention and the other therapeutic agents may have the same or different pharmaceutically effective amounts, administration times, dosing intervals, routes of administration, and treatment durations.

Another aspect of the present invention provides a method for preventing or treating cancer, comprising administering the antibody or the antigen-binding fragment thereof to a subject.

Another aspect of the present invention provides a use of the antibody or the antigen-binding fragment thereof for use in preventing or treating cancer.

Another aspect of the present invention provides use of the antibody or the antigen-binding fragment thereof in the manufacture of a composition for preventing, ameliorating, or treating cancer, preferably a pharmaceutical composition.

In the method or use for prevention or treatment according to the present invention, unless otherwise specified, the relevant terms are to be understood as having the same meanings as those described above.

In addition, in the method or use for preventing or treating cancer according to the present invention, the antibody or the antigen-binding fragment thereof may be administered to a subject simultaneously, sequentially, or separately with other therapeutic agents.

As used herein, the term "simultaneous administration" refers to administration of the antibody or the antigen-binding fragment thereof and another therapeutic agent at the same time, either as a single formulation or as separate formulations, in which case the route of administration of the antibody or the antigen-binding fragment thereof and that of the other therapeutic agent may be the same or different. In addition, the term "sequential administration" refers to administration of the antibody or the antigen-binding fragment thereof and another therapeutic agent in a relatively continuous manner, allowing only a minimal interval between administrations. Further, the term "separate administration" refers to administration of the antibody or the antigen-binding fragment thereof and another therapeutic agent at predetermined time intervals. The mode of administration of the antibody or the antigen-binding fragment thereof and the other therapeutic agent may be appropriately selected by a physician or a skilled practitioner in the art in consideration of therapeutic efficacy, side effects, and the like in the subject.

Another aspect of the present invention provides a composition for diagnosing cancer, comprising the antibody or the antigen-binding fragment thereof.

Another aspect of the present invention provides a kit for diagnosing cancer, comprising the antibody or the antigen-binding fragment thereof.

In the composition and kit for diagnosing cancer according to the present invention, unless otherwise specified, the relevant terms are to be understood as having the same meanings as those described above.

Specifically, the diagnosis of cancer according to the present invention may be performed by reacting the antibody or the antigen-binding fragment thereof with a biological sample to determine the presence or likelihood of onset of cancer, and preferably by contacting the antibody or the antigen-binding fragment thereof with the biological sample to determine whether an antigen-antibody complex is formed.

As used herein, the term "biological sample" includes, but is not limited to, tissues, cells, blood, serum, plasma, and tissue biopsy samples (e.g., brain, skin, lymph nodes, and spinal cord).

As used herein, the term "antigen-antibody complex" refers to a complex formed by binding of an HLA-G protein antigen present in a sample to the antibody or the antigen-binding fragment thereof that recognizes the antigen.

Formation of such an antigen-antibody complex may be detected by any method including, but not limited to, a colorimetric method, an electrochemical method, a fluorometric method, a luminometric method, a particle counting method, a visual assessment method, or a scintillation counting method. However, the detection is not limited thereto, and various modifications and applications are possible according to methods known in the art.

Specifically, the diagnostic composition or kit of the present invention may be prepared to be suitable for various immunoassays or immunostaining methods. The immunoassays or immunostaining methods include, but are not limited to, enzyme-linked immunosorbent assay (ELISA), immunofluorescence, Western blotting, immunohistochemistry staining, flow cytometry, immunocytochemistry, radioimmunoassay (RIA), immunoprecipitation assay, and protein chips.

In addition, labels for qualitatively or quantitatively measuring the formation of the antigen-antibody complex include, but are not limited to, enzymes, fluorescent materials, ligands, luminescent materials, microparticles, redox molecules, and radioisotopes.

Another aspect of the present invention provides a method for providing information for diagnosing cancer, comprising detecting an HLA-G protein in a biological sample isolated from a subject suspected of having cancer by an antigen-antibody reaction, or measuring the expression level or activity of the HLA-G protein, using the antibody or the antigen-binding fragment thereof.

In the method for providing information for diagnosing cancer according to the present invention, unless otherwise specified, the relevant terms are to be understood as having the same meanings as those described above.

In the present invention, the method for providing information for diagnosing cancer may be performed by reacting the antibody or the antigen-binding fragment thereof with a biological sample to provide information on the presence or likelihood of onset of cancer, and preferably by contacting the antibody or the antigen-binding fragment thereof with the biological sample to determine whether an antigen-antibody complex is formed.

In one embodiment of the present invention, the method for providing information for diagnosing cancer may be a method for diagnosing cancer, a method for providing information for selecting a cancer patient exhibiting resistance or refractoriness to an anticancer agent, or a method for selecting a cancer patient exhibiting resistance or refractoriness to an anticancer agent.

Specifically, the method may comprise (a) treating a biological sample isolated from a subject suspected of having cancer with the antibody or the antigen-binding fragment thereof to detect an HLA-G protein through an antigen-antibody reaction; and (b) comparing the expression level or activity of the HLA-G protein detected in step (a) with that of a control to determine whether the subject is a cancer patient, wherein the subject is determined to be a cancer patient when the expression level or activity of the HLA-G protein is higher than that of the control.

The control may be a biological sample isolated from a normal individual who does not have cancer or from an individual who has been cured of cancer.

Another aspect of the present invention provides a method for providing information for treating cancer, comprising detecting an HLA-G protein in a biological sample isolated from a cancer patient by an antigen-antibody reaction, or measuring the expression level or activity of the HLA-G protein, using the antibody or the antigen-binding fragment thereof.

In the method for providing information for treating cancer according to the present invention, unless otherwise specified, the relevant terms are to be understood as having the same meanings as those described above.

In the present invention, the method for providing information for treating cancer may be performed by reacting the antibody or the antigen-binding fragment thereof with a biological sample to provide information on resistance or refractoriness to an anticancer agent, and preferably by contacting the antibody or the antigen-binding fragment thereof with the biological sample to determine whether an antigen-antibody complex is formed.

In one embodiment of the present invention, the method for providing information for treating cancer may be a method for providing information for selecting a cancer patient exhibiting resistance or refractoriness to an anticancer agent, or a method for selecting a cancer patient exhibiting resistance or refractoriness to an anticancer agent.

Specifically, the method may comprise (a) treating a biological sample isolated from a cancer patient with the antibody or the antigen-binding fragment thereof to detect an HLA-G protein through an antigen-antibody reaction; and (b) comparing the expression level or activity of the HLA-G protein detected in step (a) with that of a control to determine whether the cancer is likely to be resistant or refractory to an anticancer agent, wherein the cancer is determined to be more likely to be resistant or refractory to the anticancer agent when the expression level or activity of the HLA-G protein is higher than that of the control.

The control may be a biological sample isolated from a normal individual who does not have cancer, from an individual who has been cured of cancer, or from a cancer patient determined not to exhibit resistance or refractoriness to an anticancer agent.

Another aspect of the present invention provides a method for screening an anticancer agent, comprising (a) treating cancer cells with a candidate anticancer agent; (b) measuring the expression level or activity of an HLA-G protein in the cancer cells treated with the candidate anticancer agent using the antibody or the antigen-binding fragment thereof; and (c) determining the candidate anticancer agent treated in step (a) to be an anticancer agent when the expression level or activity of the HLA-G protein in step (b) is reduced compared to that of cancer cells not treated with the candidate anticancer agent.

In the method for screening an anticancer agent according to the present invention, unless otherwise specified, the relevant terms are to be understood as having the same meanings as those described above.

Specifically, step (a) is a step of treating cancer cells with a candidate anticancer agent, and may be performed using methods known in the art. For example, the candidate anticancer agent may be applied by co-culturing the cancer cells therewith or by administering it to an in vivo system comprising cancer cells; however, the method is not limited thereto. Those skilled in the art may employ any suitable method consistent with the purpose of the present invention.

As used herein, the term "anticancer agent" refers to a substance that exhibits a preventive or therapeutic effect against cancer, and specifically refers to a substance capable of killing cancer cells or tumors or inhibiting the growth thereof. In the present specification, the term "anticancer agent" may be used interchangeably with "drug," and the term "treating" may be used interchangeably with "adding" or "administering".

As used herein, the term "candidate anticancer agent" refers to a substance expected to exhibit a preventive or therapeutic effect against cancer, and specifically refers to a substance expected to kill cancer cells or tumors or inhibit the growth thereof.

Preferably, the anticancer agent or candidate anticancer agent includes, without limitation, compounds, proteins, fusion proteins, compound-protein complexes, drug-protein complexes, antibodies, compound-antibody complexes, drug-antibody complexes, amino acids, peptides, viruses, carbohydrates, lipids, nucleic acids, extracts, and fractions. For example, it may include, but is not limited to, compounds, peptides, peptide mimetics, fusion proteins, antibodies, aptamers, and antibody-drug conjugates (ADCs). Preferably, it may be an antibody or an immuno-oncology agent.

Step (b) is a step of measuring the expression level or activity of the HLA-G protein, and any method known to those skilled in the art may be used. Specific examples include, but are not limited to, Western blotting, co-immunoprecipitation assay, enzyme-linked immunosorbent assay (ELISA), immunostaining, and flow cytometry analysis. Those skilled in the art may employ any suitable method consistent with the purpose of the present invention.

Step (c) is a step of determining whether the candidate anticancer agent can be used as an anticancer agent.

The antibody or the antigen-binding fragment thereof that specifically binds to human leukocyte antigen G (HLA-G) according to the present invention may exhibit excellent anticancer effects, such as inhibiting tumor growth by specifically binding to HLA-G and inhibiting the interaction between HLA-G and ILT-2.

### [Brief Description of Figures]

FIG. 1A shows the results of confirming, using ELISA, concentration-dependent binding of anti-HLA-G antibodies to HLA-G. FIG. 1B shows the results of confirming specific binding activity of anti-HLA-G antibodies to HLA-G.
FIG. 2 shows the results of confirming, using ELISA, the inhibitory activity of anti-HLA-G antibodies against binding between HLA-G and recombinant ILT-2 protein.
FIG. 3 shows the results of confirming, using FACS, concentration-dependent binding of anti-HLA-G antibodies to cancer cell lines naturally expressing HLA-G.
FIG. 4 shows the results of confirming, using FACS, the cancer cell killing activity induced by anti-HLA-G antibodies through inhibition of binding between HLA-G overexpressed in SKOV-3/HLA-G cells and ILT-2 naturally expressed on NK92-MI natural killer cells.
FIG. 5 shows the results of confirming, using FACS, increased cancer cell killing activity induced by anti-HLA-G antibodies that bind to HLA-G expressed in various cancer types and thereby inhibit binding between HLA-G and ILT-2 naturally expressed on NK92-MI natural killer cells.
FIG. 6 shows the results of confirming, using FACS, antibody-dependent cellular phagocytosis of anti-HLA-G antibodies mediated by macrophages.
FIG. 7 shows the results of confirming cancer cell killing activity of anti-HLA-G antibodies in an in vivo xenograft mouse model using pancreatic cancer cell lines and gastric cancer cell lines.
FIG. 8 shows the results of an in vitro assay evaluating antibody-dependent cellular cytotoxicity of anti-HLA-G antibodies and afucosylated anti-HLA-G antibodies.
FIG. 9 shows the results of confirming, using ELISA, the inhibitory activity of materials prepared based on anti-HLA-G antibodies and antigen-binding fragments thereof against binding between HLA-G and ILT-2.

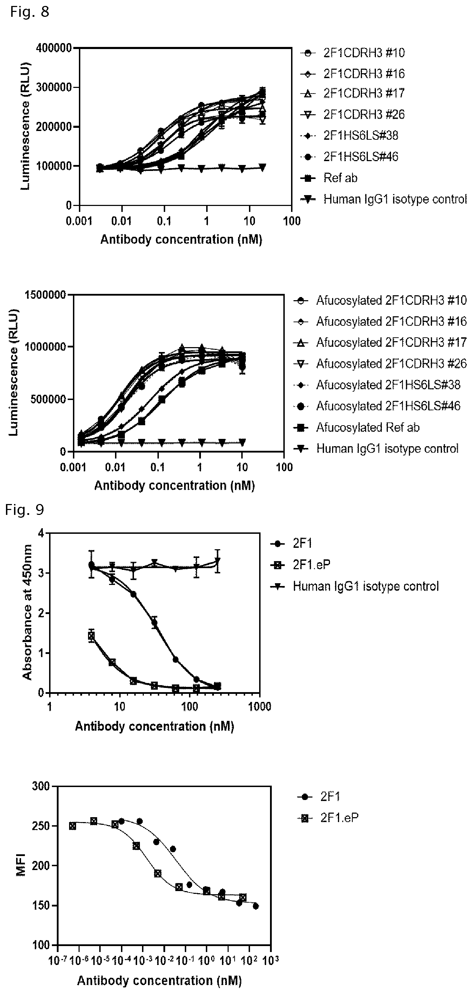

FIG. 9 shows the results of confirming, using ELISA, the inhibitory activity against binding between HLA-G and ILT-2 for materials prepared based on anti-HLA-G antibodies and antigen-binding fragments thereof.

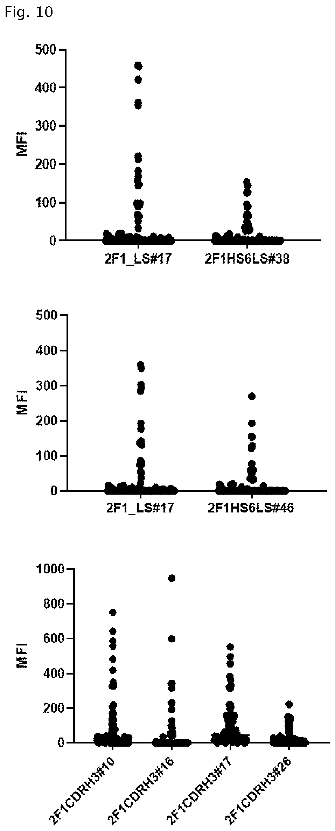

FIG. 10 shows the results of HLA subtype selectivity of anti-HLA-G antibodies.

### [Examples]

The present invention will be described in more detail with reference to the following Examples. However, these Examples are provided for illustrative purposes only, and the scope of the present invention is not limited thereto.

### Example 1. Selection of anti-HLA-G antibodies

### Example 1.1. Preparation of library phage by phage display

A human synthetic Fab library having diversity was inoculated into 2×YT medium containing glucose and cultured in a shaking incubator at 37°C until the optical density of the culture (OD600) reached 0.5 to 0.7.

To remove glucose from the medium, the culture was centrifuged (5,000 rpm, 4°C, 20 min), the supernatant was removed, and the culture was resuspended in 2×YT medium. Thereafter, helper phage was added for infection, followed by culturing in a shaking incubator at 37°C for about 1 hour. Kanamycin was then added at a concentration of 50 µg/mL, and cultured in a shaking incubator at 30°C and 120 rpm for 16 hours. The cultured cells were centrifuged (8,000 rpm, 4°C, 20 min), and the supernatant was collected, followed by addition of 4% PEG (Sigma, P5413) and 3% NaCl (Sigma, S9888), which were dissolved thoroughly, and incubation on ice for about 1 hour. The mixture was centrifuged again (12,000 rpm, 4°C, 30 min), and the resulting pellet was resuspended in DPBS (Wellgene, LB001-02), followed by centrifugation (10,000 rpm, 4°C, 10 min). The supernatant was collected, transferred to a new tube, stored at 5 ± 3°C, and used for panning.

### Example 1.2. Phage panning

To select antibodies binding to HLA-G, the HLA-G antigen was diluted in PBS to a concentration of 10 µg/mL, added to an immunotube, and incubated at 4°C overnight. For negative selection to remove phages binding only to B2M and HLA subtypes HLA-B, HLA-C and HLA-E, the antigens B2M, HLA-B, HLA-C and HLA-E were diluted in PBS to a concentration of 25 µg/mL and incubated with magnetic beads overnight. Thereafter, in order to remove antigens that were not bound to the immunotube and magnetic beads, the supernatant was removed, and 5% skim milk solution was added to the immunotube and magnetic beads to block surfaces not occupied by HLA-G, B2M, HLA-B, HLA-C, and HLA-E. The phage prepared in Example 1.1 was diluted in 5% skim milk solution, added to the magnetic beads, and allowed to bind to the B2M antigen, followed by incubation at room temperature for 1 hour. The supernatant was collected using a magnet and transferred to the immunotube coated with HLA-G, followed by incubation at room temperature for 1 hour. After 1 hour, the supernatant in the immunotube was removed, and non-specifically bound phages were washed four times with PBS-T (phosphate-buffered saline with 0.05% Tween 20), followed by two additional washes with DPBS. The remaining antigen-specific phage antibodies were recovered after adding 0.1 M glycine-HCl (pH 2.7) and incubating at room temperature for 10 minutes. The recovered phages were neutralized with 2 M Tris-HCl (pH 10.05), followed by infection into TG1 *Escherichia coli* in a shaking incubator at 37°C for 1 hour. Thereafter, centrifugation (4,000 rpm, 4°C, 30 min) was performed, the supernatant was removed, and the pellet was resuspended in 2×YT medium. The resuspended pellet was plated on 2×YT-carbenicillin plates containing 2% glucose and cultured at 37°C for about 16 hours. On the following day, 6 mL of 2×YT medium was added to the plate to resuspend the colonies, and a portion of the suspension was inoculated into 50 mL of 2×YT-carbenicillin medium and cultured in a shaking incubator at 37°C. When the optical density of the culture (OD600) reached 0.5 to 0.6, helper phage was added at an MOI of 20 and cultured at 37°C for 1 hour with gentle agitation. Thereafter, kanamycin was added at 50 µg/mL, followed by culturing in a shaking incubator at 30°C for 16 to 20 hours. On the following day, the culture was centrifuged, and the supernatant was collected. Subsequently, 4% PEG (Sigma, P5413) and 3% NaCl (Sigma, S9888) were added and allowed to precipitate at 4°C for 1 hour, followed by centrifugation. The supernatant was removed, and the precipitated phages were resuspended in 5 mL of PBS and used as a library. The above panning process was repeated four times to amplify and enrich antigen-specific clones. In this process, the amount of HLA-G coated on the immunotube was reduced by half in each round, the number of PBS-T washes was increased by one in each round, and the antigens used for negative selection were changed to HLA-B in the second round, HLA-C in the third round, and HLA-E in the fourth round.

### Example 1.3. Monoclonal phage ELISA

To select clones that specifically bind to the antigen among the phages obtained through panning in Example 1.2, a monoclonal phage ELISA was performed. After the fourth round of panning, single clones were selected from a solid medium spread with the phage, and inoculated into a 96-well deep-well plate containing 1 mL of 2×YT-carbenicillin medium per well, followed by culturing in a shaking incubator at 37°C until the optical density (OD600) reached 0.5. After cultivation, helper phage was added, followed by infection in a shaking incubator at 37°C for 1 hour. Thereafter, kanamycin was added, and culturing was continued at 30°C for 20 hours. Subsequently, centrifugation was performed to precipitate the cells, and the supernatant was collected. Binding of Fab to the HLA-G antigen was then confirmed using an ELISA method. The Fab bound to the antigen was detected using horseradish peroxidase (HRP)-conjugated anti-M13 antibody and a TMB substrate. Clones exhibiting an absorbance (OD450) of 0.4 or higher for HLA-G were selected, and the sequences of the genes were analyzed.

As a result, as shown in Table 1 below, clones 2F1 (SEQ ID NOs: 15 and 16), T22A4 (SEQ ID NOs: 31 and 32), and T23D5 (SEQ ID NOs: 47 and 48) were selected.

**[Table 1]**

| Clone | Amino acid sequence | | SEQ ID NO. |
|---|---|---|---|
| 2F1 | CDRH1 | SYAMS | 1 |
| | CDRH2 | RISGSGGYIYYADSVKG | 2 |
| | CDRH3 | EGTSSYRGWMDV | 3 |
| | CDRL1 | RASQSISNYLN | 4 |
| | CDRL2 | AASSLQS | 5 |
| | CDRL3 | CQQSYSSPWT | 6 |
| | FRH1 | EVQLVESGGGLVQPGGSLRLSCAASGFTFS | 7 |
| | FRH2 | WVRQAPGKGLEWVS | 8 |
| | FRH3 | RFTISRDNSKNTLYLQMNSLRAEDTAVYYCAK | 9 |
| | FRH4 | WGQGTLVTVSS | 10 |
| | FRL1 | DIQMTQSPSSLSASVGDRVTITC | 11 |
| | FRL2 | WYQQKPGKAPKLLIY | 12 |
| | FRL3 | GVPSRFSGSGSGTDFTLTISSLQPEDFATYY | 13 |
| | FRL4 | FGQGTKVEIK | 14 |
| | Heavy chain variable region | EVQLVESGGGLVQPGGSLRLSCAASGFTFSSYAMSW VRQAPGKGLEWVSRISGSGGYIYYADSVKGRFTISRD NSKNTLYLQMNSLRAEDTAVYYCAKEGTSSYRGWM DVWGQGTLVTVSS | 15 |
| | Light chain variable region | DIQMTQSPSSLSASVGDRVTITCRASQSISNYLNWYQQ KPGKAPKLLIYAASSLQSGVPSRFSGSGSGTDFTLTISS LQPEDFATYYCQQSYSSPWTFGQGTKVEIK | 16 |
| T22A4 | CDRH1 | SYAMS | 17 |
| | CDRH2 | SISGSGNSELYAGSVKG | 18 |
| | CDRH3 | FSAAVGFLPDYFDS | 19 |
| | CDRL1 | QASHDISKYLN | 20 |
| | CDRL2 | DASNLET | 21 |
| | CDRL3 | QQSYRTPLT | 22 |
| | FRH1 | EVQLVESGGNWVQPGGSLRLSCAASGFSFR | 23 |
| | FRH2 | WVRQAPGKGLEWVS | 24 |
| | FRH3 | RFTISRDNSKNTLSLQLHSLRAEDTAMYYCAK | 25 |
| | FRH4 | WGQGTLVTVSS | 26 |
| | FRL1 | ELTLTQSPSSLSASVGDRVTITC | 27 |
| | FRL2 | WYQQKSGKAPKLLIY | 28 |
| | FRL3 | GVPSRFSGSGSGTDFTLTISSLQPEDFATYYC | 29 |
| | FRL4 | FGGGTKLEIK | 30 |
| | Heavy chain variable region | EVQLVESGGNWVQPGGSLRLSCAASGFSFRSYAMSW VRQAPGKGLEWVSSISGSGNSELYAGSVKGRFTISRD NSKNTLSLQLHSLRAEDTAMYYCAKFSAAVGFLPDY FDSWGQGTLVTVSS | 31 |
| | Light chain variable region | ELTLTQSPSSLSASVGDRVTITCQASHDISKYLNWYQQ KSGKAPKLLIYDASNLETGVPSRFSGSGSGTDFTLTISS LQPEDFATYYCQQSYRTPLTFGGGTKLEIK | 32 |
| T23D5 | CDRH1 | RSSHYWG | 33 |
| | CDRH2 | SIYYSGITYYNPSLKS | 34 |
| | CDRH3 | HGSYDSWAGRYKWDRFDP | 35 |
| | CDRL1 | QASHDISKYLN | 36 |
| | CDRL2 | DASNLETG | 37 |
| | CDRL3 | QQSYSTPLT | 38 |
| | FRH1 | QVQLQESGPGLVKPSETLSLTCTVSGGSID | 39 |
| | FRH2 | WIRQPPGKGLEWLG | 40 |
| | FRH3 | RVTISVDTSRNQFSLTLSSVTAADTAVYYCAR | 41 |
| | FRH4 | WGQGTPVTVSS | 42 |
| | FRL1 | ELQMTQSPSSLSASVGDRVTITC | 43 |
| | FRL2 | WYQQKSGKAPKLLIY | 44 |
| | FRL3 | VPSRFSGSGSGTDFTLTISSLQPEDFATYYC | 45 |
| | FRL4 | FGGGTKVEIK | 46 |
| | Heavy chain variable region | QVQLQESGPGLVKPSETLSLTCTVSGGSIDRSSHYWG WIRQPPGKGLEWLGSIYYSGITYYNPSLKSRVTISVDT SRNQFSLTLSSVTAADTAVYYCARHGSYDSWAGRYK WDRFDPWGQGTPVTVSS | 47 |
| | Light chain variable region | ELQMTQSPSSLSASVGDRVTITCQASHDISKYLNWYQ QKSGKAPKLLIYDASNLETGVPSRFSGSGSGTDFTLTI SSLQPEDFATYYCQQSYSTPLTFGGGTKVEIK | 48 |

### Example 1.4. Antibody optimization

To obtain various antibodies binding to HLA-G and to optimize their efficacy, various types of shuffling libraries were constructed as described below, and antibodies were screened using the same method as in Example 1.2.

To construct a 2F1 light chain shuffling library, the heavy chain of clone 2F1 selected from the library of Example 1.1 was fixed, and CDRL1, CDRL2 and CDRL3 derived from a human synthetic Fab library were introduced to construct a light chain shuffling library in the form of scFv. Thereafter, antibody screening was performed using the same methods as in Examples 1.2 and 1.3.

As a result, as shown in Table 2 below, 2F1_LS#17 (SEQ ID NOs: 15 and 63), 2F1_LS#24 (SEQ ID NOs: 15 and 78), 2F1_LS#26 (SEQ ID NOs: 15 and 93), and 2F1_LS#39 (SEQ ID NOs: 15 and 108) were selected.

**[Table 2]**

| Clone | Amino acid sequence | | SEQ ID NO. |
|---|---|---|---|
| 2F1_LS#17 | CDRH1 | SYAMS | 49 |
| | CDRH2 | RISGSGGYIYYADSVKG | 50 |
| | CDRH3 | EGTSSYRGWMDV | 51 |
| | CDRL1 | RASQSISNYLN | 52 |
| | CDRL2 | AASRLQS | 53 |
| | CDRL3 | QQSYSTPWT | 54 |
| | FRH1 | EVQLVESGGGLVQPGGSLRLSCAASGFTFS | 55 |
| | FRH2 | WVRQAPGKGLEWVS | 56 |
| | FRH3 | RFTISRDNSKNTLYLQMNSLRAEDTAVYYCAK | 57 |
| | FRH4 | WGQGTLVTVSS | 58 |
| | FRL1 | DIQMTQSPSSLSASVGDRVTITC | 59 |
| | FRL2 | WYQQKPGKAPKLLIY | 60 |
| | FRL3 | DVPSRFSGSGSGTDFTLTISSLQPEDFATYYC | 61 |
| | FRL4 | FGQGTKVEIK | 62 |
| | Light chain variable region | DIQMTQSPSSLSASVGDRVTITCRASQSISNYLNW YQQKPGKAPKLLIYAASRLQSDVPSRFSGSGSGTD FTLTISSLQPEDFATYYCQQSYSTPWTFGQGTKVE IK | 63 |
| 2F1_LS#24 | CDRH1 | SYAMS | 64 |
| | CDRH2 | RISGSGGYIYYADSVKG | 65 |
| | CDRH3 | EGTSSYRGWMDV | 66 |
| | CDRL1 | RASQSISNWLN | 67 |
| | CDRL2 | AASRLQS | 68 |
| | CDRL3 | QQSYTSPWT | 69 |
| | FRH1 | EVQLVESGGGLVQPGGSLRLSCAASGFTFS | 70 |
| | FRH2 | WVRQAPGKGLEWVS | 71 |
| | FRH3 | RFTISRDNSKNTLYLQMNSLRAEDTAVYYCAK | 72 |
| | FRH4 | WGQGTLVTVSS | 73 |
| | FRL1 | DIQMTQSPSSLSASVGDRVTITC | 74 |
| | FRL2 | WYQQKPGKAPKLLIY | 75 |
| | FRL3 | GVPSRFSGSGSGTDFTLTISSLQPEDFATYYC | 76 |
| | FRL4 | FGQGTKVEIK | 77 |
| | Light chain variable region | DIQMTQSPSSLSASVGDRVTITCRASQSISNWLNW YQQKPGKAPKLLIYAASRLQSGVPSRFSGSGSGTD FTLTISSLQPEDFATYYCQQSYTSPWTFGQGTKVE IK | 78 |
| 2F1_LS#26 | CDRH1 | SYAMS | 79 |
| | CDRH2 | RISGSGGYIYYADSVKG | 80 |
| | CDRH3 | EGTSSYRGWMDV | 81 |
| | CDRL1 | RASQSISNYLN | 82 |
| | CDRL2 | ATSRLQS | 83 |
| | CDRL3 | CQQSYSFPWT | 84 |
| | FRH1 | EVQLVESGGGLVQPGGSLRLSCAASGFTFS | 85 |
| | FRH2 | WVRQAPGKGLEWVS | 86 |
| | FRH3 | RFTISRDNSKNTLYLQMNSLRAEDTAVYYCAK | 87 |
| | FRH4 | WGQGTLVTVSS | 88 |
| | FRL1 | DIQMTQSPSSLSASVGDRVTITC | 89 |
| | FRL2 | WYQQKPGKAPKLLIY | 90 |
| | FRL3 | GVPSRFSGSGSGTDFTLTISSLQPEDFATYY | 91 |
| | FRL4 | FGQGTKVEIK | 92 |
| | Light chain variable region | DIQMTQSPSSLSASVGDRVTITCRASQSISNYLNW YQQKPGKAPKLLIYATSRLQSGVPSRFSGSGSGTD FTLTISSLQPEDFATYYCQQSYSFPWTFGQGTKVE IK | 93 |
| 2F1_LS#39 | CDRH1 | SYAMS | 94 |
| | CDRH2 | RISGSGGYIYYADSVKG | 95 |
| | CDRH3 | EGTSSYRGWMDV | 96 |
| | CDRL1 | RASQSISRYLN | 97 |
| | CDRL2 | ATSNLHS | 98 |
| | CDRL3 | CQQSYTSPWT | 99 |
| | FRH1 | EVQLVESGGGLVQPGGSLRLSCAASGFTFS | 100 |
| | FRH2 | WVRQAPGKGLEWVS | 101 |
| | FRH3 | RFTISRDNSKNTLYLQMNSLRAEDTAVYYCAK | 102 |
| | FRH4 | WGQGTLVTVSS | 103 |
| | FRL1 | DIQMTQSPSSLSASVGDRVTITC | 104 |
| | FRL2 | WYQQKPGKAPKLLIY | 105 |
| | FRL3 | GVPSRFSGSGSGTDFTLTISSLQPEDFATYY | 106 |
| | FRL4 | FGQGTKVEIK | 107 |
| | Light chain variable region | DIQMTQSPSSLSASVGDRVTITCRASQSISRYLNW YQQKPGKAPKLLIYATSNLHSGVPSRFSGSGSGTD FTLTISSLQPEDFATYYCQQSYTSPWTFGQGTKVE IK | 108 |

Next, in order to construct a 2F1_LS#17 CDRH1 and CDRH2, and CDRL1, CDRL2 and CDRL3 shuffling library, CDRH3 of 2F1_LS#17 was fixed, and CDRH1, CDRH2, CDRL1, CDRL2 and CDRL3 derived from a human synthetic Fab library were introduced to construct a light chain and heavy chain shuffling library in the form of scFv. Thereafter, antibody screening was performed using the same methods as in Examples 1.1 to 1.3.

As a result, as shown in Table 3 below, 2F1HS6LS#38 (SEQ ID NOs: 123 and 124) and 2F1HS6LS#46 (SEQ ID NOs: 123 and 139) were selected.

**[Table 3]**

| Clone | Amino acid sequence | | SEQ ID NO. |
|---|---|---|---|
| 2F1HS6LS#38 | CDRH1 | DYAMS | 109 |
| | CDRH2 | RISQSGSYTYYADSVKG | 110 |
| | CDRH3 | EGTSSYRGWMDV | 111 |
| | CDRL1 | RASQSISSYLN | 112 |
| | CDRL2 | AASRLQS | 113 |
| | CDRL3 | QQSYSTPWT | 114 |
| | FRH1 | EVQLVESGGGLVQPGGSLRLSCAASGFTFS | 115 |
| | FRH2 | WVRQAPGKGLEWVS | 116 |
| | FRH3 | RFTISRDNSKNTLYLQMNSLRAEDTAVYYCAK | 117 |
| | FRH4 | WGQGTLVTVSS | 118 |
| | FRL1 | DIQMTQSPSSLSASVGDRVTITC | 119 |
| | FRL2 | WYQQKPGKAPKLLIY | 120 |
| | FRL3 | GVPSRFSGSGSGTDFTLTISSLQPEDFATYYC | 121 |
| | FRL4 | FGQGTKVEIK | 122 |
| | Heavy chain variable region | EVQLVESGGGLVQPGGSLRLSCAASGFTFSDY AMSWVRQAPGKGLEWVSRISQSGSYTYYADS VKGRFTISRDNSKNTLYLQMNSLRAEDTAVYY CAKEGTSSYRGWMDVWGQGTLVTVSS | 123 |
| | Light chain variable region | DIQMTQSPSSLSASVGDRVTITCRASQSISSYLN WYQQKPGKAPKLLIYAASRLQSGVPSRFSGSG SGTDFTLTISSLQPEDFATYYCQQSYSTPWTFG QGTKVEIK | 124 |
| 2F1HS6LS#46 | CDRH1 | DYAMS | 125 |
| | CDRH2 | RISQSGSYTYYADSVKG | 126 |
| | CDRH3 | EGTSSYRGWMDV | 127 |
| | CDRL1 | RASQSISNWLN | 128 |
| | CDRL2 | ATSRLQS | 129 |
| | CDRL3 | QQSYSTPWT | 130 |
| | FRH1 | EVQLVESGGGLVQPGGSLRLSCAASGFTFS | 131 |
| | FRH2 | WVRQAPGKGLEWVS | 132 |
| | FRH3 | RFTISRDNSKNTLYLQMNSLRAEDTAVYYCAK | 133 |
| | FRH4 | WGQGTLVTVSS | 134 |
| | FRL1 | DIQMTQSPSSLSASVGDRVTITC | 135 |
| | FRL2 | WYQQKPGKAPKLLIY | 136 |
| | FRL3 | GVPSRFSGSGSGTDFTLTISSLQPEDFATYYC | 137 |
| | FRL4 | FGQGTKVEIK | 138 |
| | Light chain variable region | DIQMTQSPSSLSASVGDRVTITCRASQSISNWL NWYQQKPGKAPKLLIYATSRLQSGVPSRFSGS GSGTDFTLTISSLQPEDFATYYCQQSYSTPWTF GQGTKVEIK | 139 |

Next, in order to construct a 2F1 CDRH1 and CDRH2 shuffling, CDRH3 mutagenesis, and CDRL1, CDRL2 and CDRL3 shuffling library, mutations were introduced into CDRH3 of 2F1_LS#17 using an NNK method, and CDRH1, CDRH2, CDRL1, CDRL2 and CDRL3 derived from a human synthetic Fab library were introduced to construct a light chain and heavy chain shuffling library in the form of scFv. Thereafter, antibody screening was performed using the same methods as in Examples 1.1 to 1.3. As a result, as shown in Table 4 below, 2F1CDRH3#10 (SEQ ID NOs: 154 and 155), 2F1CDRH3#16 (SEQ ID NOs: 170 and 171), 2F1CDRH3#17 (SEQ ID NOs: 186 and 187), and 2F1CDRH3#26 (SEQ ID NOs: 202 and 203) were selected.

**[Table 4]**

| Clone | Amino acid sequence | | SEQ ID NO. |
|---|---|---|---|
| 2F1CDRH3#10 | CDRH1 | SYAMS | 140 |
| | CDRH2 | RISGSGGYKYYADSVKG | 141 |
| | CDRH3 | EGTSSYRGWMDV | 142 |
| | CDRL1 | RASQSISNYLN | 143 |
| | CDRL2 | ATSTLQS | 144 |
| | CDRL3 | QQSYTTPWT | 145 |
| | FRH1 | EVQLVESGGGLVQPGGSLRLSCAASGFTFS | 146 |
| | FRH2 | WVRQAPGKGLEWVS | 147 |
| | FRH3 | RFTISRDNSKNTLYLQMNSLRAEDTAVYYCAK | 148 |
| | FRH4 | WGQGTLVTVSS | 149 |
| | FRL1 | DIQMTQSPSSLSASVGDRVTITC | 150 |
| | FRL2 | WYQQKPGKAPKLLIY | 151 |
| | FRL3 | GVPSRFSGSGSGTDFTLTISSLQPEDFATYYC | 152 |
| | FRL4 | FGQGTKVEIK | 153 |
| | Heavy chain variable region | EVQLVESGGGLVQPGGSLRLSCAASGFTFSSYA MSWVRQAPGKGLEWVSRISGSGGYKYYADSV KGRFTISRDNSKNTLYLQMNSLRAEDTAVYYC AKEGTSSYRGWMDVWGQGTLVTVSS | 154 |
| | Light chain variable region | IQMTQSPSSLSASVGDRVTITCRASQSISNYLN WYQQKPGKAPKLLIYATSTLQSGVPSRFSGSGS GTDFTLTISSLQPEDFATYYCQQSYTTPWTFGQ GTKVEIK | 155 |
| 2F1CDRH3#16 | CDRH1 | DYAMS | 156 |
| | CDRH2 | RISSSGSYTYYADSVKG | 157 |
| | CDRH3 | EGTSKYRGWMDV | 158 |
| | CDRL1 | RASQSISNYLN | 159 |
| | CDRL2 | AASTLQS | 160 |
| | CDRL3 | QQSYSTPWT | 161 |
| | FRH1 | EVQLVESGGGLVQPGGSLRLSCAASGFTFS | 162 |
| | FRH2 | WVRQAPGKGLEWVS | 163 |
| | FRH3 | RFTISRDNSKNTLYLQMNSLRAEDTAVYYCAK | 164 |
| | FRH4 | WGQGTLVTVSS | 165 |
| | FRL1 | DIQMTQSPSSLSASVGDRVTITC | 166 |
| | FRL2 | WYQQKPGKAPKLLIY | 167 |
| | FRL3 | GVPSRFSGSGSGTDFTLTISSLQPEDFATYYC | 168 |
| | FRL4 | FGQGTKVEIK | 169 |
| | Heavy chain variable region | EVQLVESGGGLVQPGGSLRLSCAASGFTFSDY AMSWVRQAPGKGLEWVSRISSSGSYTYYADS VKGRFTISRDNSKNTLYLQMNSLRAEDTAVYY CAKEGTSKYRGWMDVWGQGTLVTVSS | 170 |
| | Light chain variable region | DIQMTQSPSSLSASVGDRVTITCRASQSISNYLN WYQQKPGKAPKLLIYAASTLQSGVPSRFSGSGS GTDFTLTISSLQPEDFATYYCQQSYSTPWTFGQ GTKVEIK | 171 |
| 2F1CDRH3#17 | CDRH1 | DYAMS | 172 |
| | CDRH2 | RISGTGSYKYYADSVKG | 173 |
| | CDRH3 | EGTSTYRGWMDV | 174 |
| | CDRL1 | RASQSISNYLN | 175 |
| | CDRL2 | ATSRLQS | 176 |
| | CDRL3 | QQSYTTPWT | 177 |
| | FRH1 | EVQLVESGGGLVQPGGSLRLSCAASGFTFS | 178 |
| | FRH2 | WVRQAPGKGLEWVS | 179 |
| | FRH3 | RFTISRDNSKNTLYLQMNSLRAEDTAVYYCAK | 180 |
| | FRH4 | WGQGTLVTVSS | 181 |
| | FRL1 | DIQMTQSPSSLSASVGDRVTITC | 182 |
| | FRL2 | WYQQKPGKAPKLLIY | 183 |
| | FRL3 | GVPSRFSGSGSGTDFTLTISSLQPEDFATYYC | 184 |
| | FRL4 | FGQGTKVEIK | 185 |
| | Heavy chain variable region | EVQLVESGGGLVQPGGSLRLSCAASGFTFSDY AMSWVRQAPGKGLEWVSRISGTGSYKYYADS VKGRFTISRDNSKNTLYLQMNSLRAEDTAVYY CAKEGTSTYRGWMDVWGQGTLVTVSS | 186 |
| | Light chain variable region | DIQMTQSPSSLSASVGDRVTITCRASQSISNYLN WYQQKPGKAPKLLIYATSRLQSGVPSRFSGSGS GTDFTLTISSLQPEDFATYYCQQSYTTPWTFGQ GTKVEIK | 187 |
| 2F1CDRH3#26 | CDRH1 | SYAMS | 188 |
| | CDRH2 | RISGSGSYTYYADSVKG | 189 |
| | CDRH3 | EGASSYRGWMDV | 190 |
| | CDRL1 | ASQSISNYLN | 191 |
| | CDRL2 | ATSRLQS | 192 |
| | CDRL3 | QQSHSTPWT | 193 |
| | FRH1 | EVQLVESGGGLVQPGGSLRLSCAASGFTFS | 194 |
| | FRH2 | WVRQAPGKGLEWVS | 195 |
| | FRH3 | RFTISRDNSKNTLYLQMNSLRAEDTAVYYCAK | 196 |
| | FRH4 | WGQGTLVTVSS | 197 |
| | FRL1 | DIQMTQSPSSLSASVGDRVTITCR | 198 |
| | FRL2 | WYQQKPGKAPKLLIY | 199 |
| | FRL3 | GVPSRFSGSGSGTDFTLTISSLQPEDFATYYC | 200 |
| | FRL4 | FGQGTKVEIK | 201 |
| | Heavy chain variable region | EVQLVESGGGLVQPGGSLRLSCAASGFTFSSYA MSWVRQAPGKGLEWVSRISGSGSYTYYADSV KGRFTISRDNSKNTLYLQMNSLRAEDTAVYYC AKEGASSYRGWMDVWGQGTLVTVSS | 202 |
| | Light chain variable region | DIQMTQSPSSLSASVGDRVTITCRASQSISNYLN WYQQKPGKAPKLLIYATSRLQSGVPSRFSGSGS GTDFTLTISSLQPEDFATYYCQQSHSTPWTFGQ GTKVEIK | 203 |

### Example 2. Preparation of anti-HLA-G antibody

Based on the sequences of the antibody variable regions obtained in Examples 1.2 to 1.4 and the sequences of the positive control antibody (Fab and scFv) described in Table 5 below, an anti-HLA-G antibody was prepared by linking the heavy chain variable region to the heavy chain constant region (SEQ ID NO.: 220) described in Table 5 and linking the light chain variable region to the light chain constant region (SEQ ID NO.: 221) described in Table 5. The positive control antibody is an antibody that selectively binds to HLA-G and comprises the sequences of SEQ ID NOs: 238 and 306 disclosed in US 2020/0102390 A1. In the drawings attached to the present specification, "Ref ab" indicates the positive control antibody.

To prepare the 2F1.eP antibody, the heavy chain variable region (SEQ ID NO.: 15) was linked to IgD hinge+IgG1+IgM (SEQ ID NO.: 222).

**[Table 5]**

| Clone | Amino acid sequence | | SEQ ID NO. |
|---|---|---|---|
| Positive control antibody | CDRH1 | SSSTYWS | 204 |
| | CDRH2 | GIAYSGSTYYNPSLKS | 205 |
| | CDRH3 | GVRRAVPEDY | 206 |
| | CDRL1 | QAVSSNYLA | 207 |
| | CDRL2 | GASSRAT | 208 |
| | CDRL3 | QQVVHSPYT | 209 |
| | FRH1 | QLQLQESGPGLVKPSETLSLTCTVSGGSIS | 210 |
| | FRH2 | WIRQPPGKGLEWIG | 211 |
| | FRH3 | RVTISVDTSKNQFSLKLSSVTAADTAVYYCAR | 212 |
| | FRH4 | WGQGTLVTVSS | 213 |
| | FRL1 | EIVLTQSPGTLSLSPGERATLSCQAS | 214 |
| | FRL2 | WYQQKPGQAPRLLIY | 215 |
| | FRL3 | GIPDRFSGSGSGTDFTLTISRLEPEDFAVYYC | 216 |
| | FRL4 | FGGGTKVEIK | 217 |
| | Heavy chain variable region | QLQLQESGPGLVKPSETLSLTCTVSGGSISSSSTYWS WIRQPPGKGLEWIGGIAYSGSTYYNPSLKSRVTISV DTSKNQFSLKLSSVTAADTAVYYCARGVRRAVPE DYWGQGTLVTVSS | 218 |
| | Light chain variable region | EIVLTQSPGTLSLSPGERATLSCQASQAVSSNYLAW YQQKPGQAPRLLIYGASSRATGIPDRFSGSGSGTDF TLTISRLEPEDFAVYYCQQVVHSPYTFGGGTKVEIK | 219 |
| | Heavy chain constant region (IgG1) | | 220 |
| | Light chain constant region | | 221 |
| | IgD hinge+IgG1+IgM | ASTKGPSVFPLAPSSKSTSGGTAALGCLVKDYFPEP VTVSWNSGALTSGVHTFPAVLQSSGLYSLSSVVTV PSSSLGTQTYICNVNHKPSNTKVDKKVEPKSCRNT GRGGEEKKKEKEKEEQEERETKTPECPSHTQPLGV DKTHTCPPCPAPELLGGPSVFLFPPKPKDTLMISRTP EVTCVVVDVSHEDPEVKFNWYVDGVEVHNAKTK PREEQYNSTYRVVSVLTVLHQDWLNGKEYKCKVS NKALPAPIEKTISKAKGQPREPQVYTLPPSRDELTK NQVSLTCLVKGFYPSDIAVEWESNGQPENNYKTTP PVLDSDGSFFLYSKLTVDKSRWQQGNVFSCSVMH EALHNHYTQKSLSLSPGKQDTAIRVFAIPPSFASIFL TKSTKLTCLVTDLTTYDSVTISWTRQNGEAVKTHT NISESHPNATFSAVGEASICEDDWNSGERFTCTVTH TDLPSPLKQTISRPKGVALHRPDVYLLPPAREQLNS RESATITCLVTGFSPADVFVQWMQRGQPLSPEKYV TSAPMPEPQAPGRYFAHSILTVSEEEWNTGETYTC VVAHEALPNRVTERTVDKSTGKPTLYNVSLVMSD TAGTCY | 222 |
| | J chain (R106W) | QEDERIVLVDNKCKCARITSRIIRSSEDPNEDIVERNI RIIVPLNNRENISDPTSPLRTRFVYHLSDLCKKCDPT EVELDNQIVTATQSNICDEDSATETCYTYDWNKCY TAVVPLVYGGETKMVETALTPDACYPD | 223 |

Thereafter, the anti-HLA-G antibody and 2F1.eP were produced using a pcDNA3.1 expression vector (Invitrogen) and Expi293F cells. The transfected Expi293F cells were cultured in suspension at 37°C under 8% CO₂, and Expi293F expression medium was used for culturing. For expression, the heavy chain and light chain were mixed at a ratio of 1:1 according to the manual of the ExpiFectamine 293 Transfection Kit. For 2F1.eP, a J chain (SEQ ID NO.: 223) was added, followed by purification. After transfection, culturing was carried out for 5 days, and the supernatant was collected and used for purification. The antibody was purified using a MabSelect Sure (Sartorius) column, followed by buffer exchange to PBS (pH 7.4) using Amicon Ultra 15 (Millipore, UFC903024), and then used for analysis of the anti-HLA-G antibody.

### Example 3. Analysis of binding affinity of anti-HLA-G antibodies

The binding affinity of the anti-HLA-G antibodies prepared in Example 2 for the HLA-G antigen was measured using an Octet system (Sartorius). The anti-HLA-G antibodies were immobilized on an Anti-human IgG Fc Capture biosensor at a concentration of 1.5 µg/mL, and the HLA-G antigen was serially diluted two-fold from 100 nM to 1.56 nM and then added to induce an antigen-antibody reaction. Based on the results of the antigen-antibody reaction, the association rate constant (kon) and dissociation rate constant (koff) were determined, and the affinity was calculated. The results are shown in Table 6 below.

As a result, 2F1 and 2F1_LS#17, 2F1_LS#24, 2F1_LS#26, and 2F1_LS#39 selected from the light chain shuffling library of 2F1, all exhibited reliable affinities (KD) in the range of 0.5 to 1.4 nM, comparable to that of the positive control antibody.

**[Table 6]**

| | KD (M) | KD Error | ka (1/Ms) | ka Error | kdis (1/s) | kdis Error | Full R² |
|---|---|---|---|---|---|---|---|
| 2F1 | 5.232x10⁻¹⁰ | 4.911x10⁻¹² | 2.479x10⁻⁵ | 5.335x10⁻² | 1.297x10⁻⁴ | 1.185x10⁻⁶ | 0.9927 |
| 2F1_LS#17 | 6.893x10⁻¹⁰ | 4.733x10⁻¹² | 2.006x10⁻⁵ | 3.314x10⁻² | 1.383x10⁻⁴ | 9.213x10⁻⁷ | 0.9958 |
| 2F1_LS#24 | 5.533x10⁻¹⁰ | 4.343x10⁻¹² | 2.175x10⁻⁵ | 3.611x10⁻² | 1.203x10⁻⁴ | 9.229x10⁻⁷ | 0.9957 |
| 2F1_LS#26 | 6.983x10⁻¹⁰ | 3.596x10⁻¹¹ | 4.034x10⁻⁴ | 1.237x10⁻² | 2.817x10⁻⁵ | 1.448x10⁻⁶ | 0.994 |
| 2F1_LS#39 | 5.669x10⁻¹⁰ | 4.749x10⁻¹² | 1.873x10⁻⁵ | 2.929x10⁻² | 1.062x10⁻⁴ | 8.737x10⁻⁷ | 0.9964 |
| Positive control antibody | 1.359x10⁻⁹ | 1.402x10⁻¹¹ | 1.156x10⁻⁵ | 3.293x10⁻² | 1.571x10⁻⁴ | 1.557x10⁻⁶ | 0.9875 |

### Example 4. Evaluation of the efficacy of anti-HLA-G antibodies

### Example 4.1. Analysis of binding ability to recombinant HLA-G

The antigen-binding activity of the selected anti-HLA-G antibodies prepared in Example 2 was evaluated using ELISA. Recombinant HLA-G antigen was diluted in 1× PBS to a concentration of 1 µg/mL and coated onto an immunoplate (Thermo Scientific, 442404) at 50 ng/50 µL/well, followed by incubation at 4°C overnight. After washing three times, blocking was carried out with 3% skim milk in PBST at 37°C for 1 hour. After washing three times, the anti-HLA-G antibodies were serially diluted four-fold from 20 nM to prepare seven concentrations, added at 50 µL/well, and incubated at 37°C for 1 hour. After washing three times, anti-human IgG HRP was diluted 1:5,000 in blocking buffer, added at 50 µL/well, and incubated at 37°C for 1 hour. After washing three times, TMB (Sigma, T0440) was added at 50 µL/well and color development was allowed to proceed for about 10 minutes. Thereafter, 1 N H₂SO₄ was added at 50 µL/well, and absorbance was measured at 450 nm using a microplate reader.

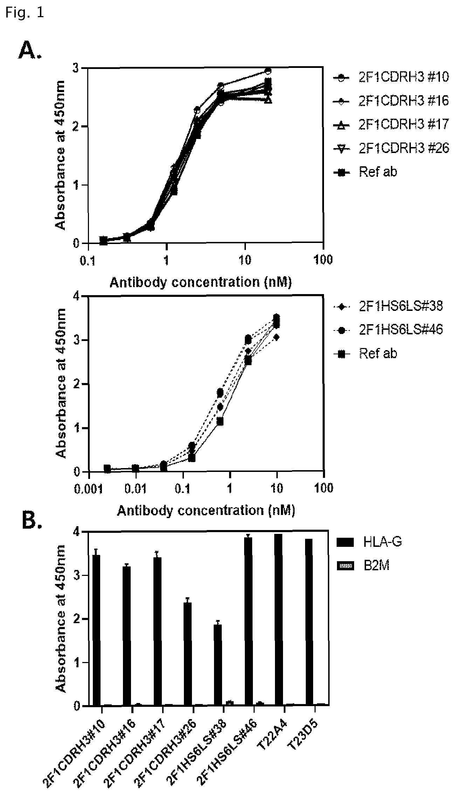

As a result, as shown in FIG. 1, all antibodies prepared in Example 2 bound to HLA-G in a concentration-dependent manner at a level comparable to that of the positive control antibody. In addition, no binding to B2M was observed, confirming specific binding to HLA-G.

### Example 4.2. Analysis of inhibitory activity of HLA-G and ILT-2 binding

To evaluate whether the anti-HLA-G antibodies prepared in Example 2 bind to the HLA-G antigen and inhibit its binding to recombinant ILT-2 protein (R&D Systems, 2017-T2), the inhibitory activity against binding between HLA-G and ILT-2 was measured using ELISA. Recombinant ILT-2_hFc (R&D Systems) was diluted in 1× PBS to a concentration of 1 µg/mL and coated onto an immunoplate (Thermo Scientific, 442404) at 100 ng/100 µL/well, followed by incubation at 4°C overnight. After washing three times, blocking was carried out with 3% skim milk in PBST at 37°C for 1 hour. After washing three times, 50 µL of HLA-G diluted in 1× PBS to a concentration of 1.2 µM and 50 µL of the test antibody serially diluted four-fold from 2,000 µM were mixed and added to each well (100 µL/well), followed by incubation at 37°C for 1 hour. After washing three times, anti-His HRP was diluted 1:10,000 in blocking buffer, added at 100 µL/well, and incubated at 37°C for 1 hour. After washing three times, TMB (Sigma, T0440) was added at 50 µL/well and color development was allowed to proceed for about 10 minutes. Thereafter, 1 N H₂SO₄ was added at 50 µL/well, and absorbance was measured at 450 nm using a microplate reader.

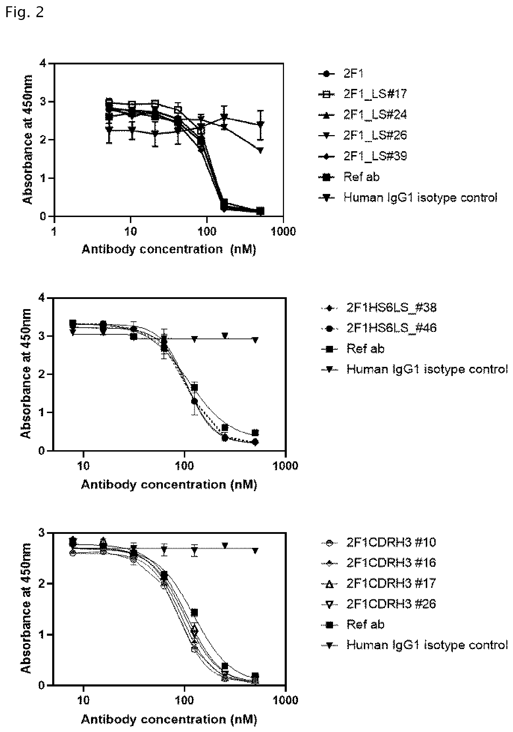

As a result, as shown in FIG. 2, the antibodies prepared in Example 2 exhibited inhibitory activity against binding between recombinant HLA-G protein and recombinant ILT-2 protein at a level comparable to that of the positive control antibody.

### Example 4.3. Analysis of cell surface antigen binding

To evaluate whether the antibodies prepared in Example 2 bind to HLA-G naturally expressed on the cell surface, a cell surface binding assay was performed. Cancer cell lines naturally expressing HLA-G, namely JEG-3 (choriocarcinoma) and SCH (gastric cancer) cells, were cultured in chemically defined medium, and then dispensed into 1.7 mL microtubes at 2×10⁵ cells/tube. Each antibody was serially diluted to six concentrations and added to the cells, followed by incubation at 4°C for 1 hour. To confirm specific binding to HLA-G on the cell surface, a human IgG1 isotype control antibody (BioXcell, BE2097) was treated in the same manner. After washing with FACS buffer, anti-human IgG PE (BioLegend) was added to each tube and incubated at 4°C for 45 minutes. The cells were then washed with FACS buffer and analyzed using FACSLyric^{™}.

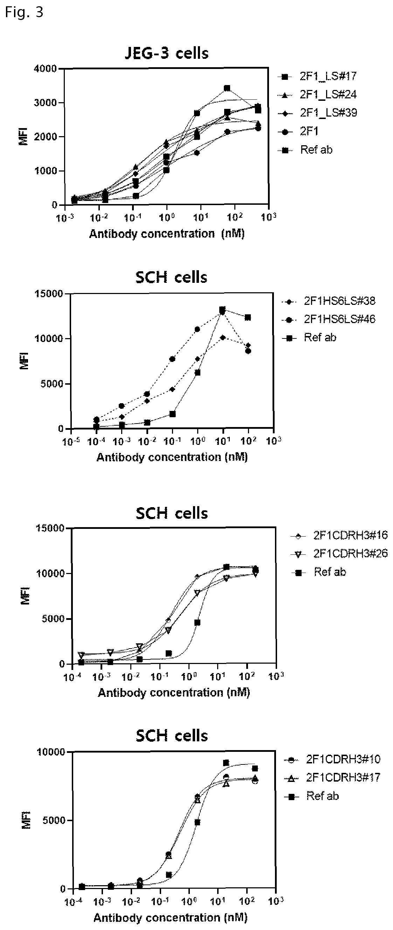

As a result, as shown in FIG. 3, among the antibodies prepared in Example 2, 2F1 and 2F1_LS#17 exhibited cell surface antigen-binding activity comparable to that of the positive control antibody. In addition, antibodies selected through antibody optimization, namely 2F1HS6LS#38, 2F1HS6LS#46, 2F1CDRH3#10, 2F1CDRH3#16, 2F1CDRH3#17, and 2F1CDRH3#26, exhibited HLA-G binding activity at least three-fold higher than that of the positive control antibody.

### Example 4.4. Evaluation of antibody-dependent cellular cytotoxicity

To determine whether the antibodies selected in Example 2 bind to HLA-G-overexpressing SKOV-3/HLA-G cells and induce Fc-mediated cytotoxicity, an antibody-dependent cellular cytotoxicity assay was performed using an ADCC reporter assay kit (Promega).

SKOV-3/HLA-G cells were plated in a 96-well white plate at 5×10³ cells/well and cultured in a 37°C, 5% CO₂ incubator for 20 hours. After removing the culture medium from the plate, ADCC effector medium (RPMI 1640 + 4% low IgG FBS) was added at 25 µL/well. Antibodies were serially diluted three-fold from 20 nM in ADCC effector medium, added to the SKOV-3/HLA-G plate at 25 µL/well, and the plate was covered and left in a clean bench. ADCC reporter cells provided in the kit were collected and resuspended in ADCC effector medium at 4×10⁶ cells/mL, and then added to the plate containing SKOV-3/HLA-G cells and antibodies at 25 µL/well, followed by incubation at 37°C under 5% CO₂ for 6 hours. The plate was then taken out and left at room temperature for 15 minutes. After that, luciferase substrate was added at 75 µL/well and allowed to react for 15 minutes in the dark. Luminescence was measured using a luminometer.

As a result, as shown in FIG. 8, the antibodies selected in Example 2 bound to HLA-G on the cell surface and exhibited antibody-dependent cellular cytotoxicity in a concentration-dependent manner. In addition, 2F1HS6LS#38, 2F1HS6LS#46, 2F1CDRH3#10, 2F1CDRH3#16, 2F1CDRH3#17, and 2F1CDRH3#26 all exhibited antibody-dependent cellular cytotoxicity comparable to or higher than that of the positive control antibody.

### Example 4.5. Analysis of enhanced cancer cell killing by natural killer cells

To determine whether the anti-HLA-G antibodies selected in Example 2 bind to HLA-G on SKOV-3/HLA-G cells and inhibit its binding to ILT-2 expressed on natural killer cells (NK92-MI), thereby increasing cancer cell killing, an analysis of HLA-G-overexpressing SKOV-3 cell killing by NK92-MI cells was performed. SKOV-3/HLA-G cells treated according to the manufacturer's instructions of a CellTrace Violet cell proliferation kit (Thermo Fisher Scientific) were seeded in a 96-well plate at 1×10⁴ cells/well. Anti-HLA-G antibodies and a human IgG1 isotype control antibody (negative control) were serially diluted five-fold from 50 nM, added at 50 µL/well, and incubated at 37°C under 5% CO₂ for 1 hour. After 1 hour, NK92-MI cells were dispensed into the wells containing SKOV-3/HLA-G cells and antibodies at 5×10⁴ cells/well, and incubated at 37°C under 5% CO₂ for 24 hours. Thereafter, TrypLE^{™} Express (Gibco) was added, and the cells in each well were transferred to FACS tubes, washed with FACS buffer (1,000 rpm, 3 minutes), and the supernatant was removed. A 7-AAD staining solution diluted 1:20 in FACS buffer was added at 100 µL/tube, followed by staining at 4°C for 20 minutes, and analysis was performed using FACSLyric^{™}. To gate only the target cells stained with CellTrace Violet from the mixed population of target and effector cells, BV420-positive cells within the overall FSC/SSC distribution were gated. To determine the proportion of dead cells among the gated target cells, only the 7-AAD-positive cell population was gated, and the ratio of 7-AAD-positive cells (P3) to target cells (P2) was calculated (% lysis = [number of P3 cells]/[number of P2 cells] × 100). Since spontaneous lysis of each target cell population was less than 5%, it was not included in the calculation of cytotoxicity.

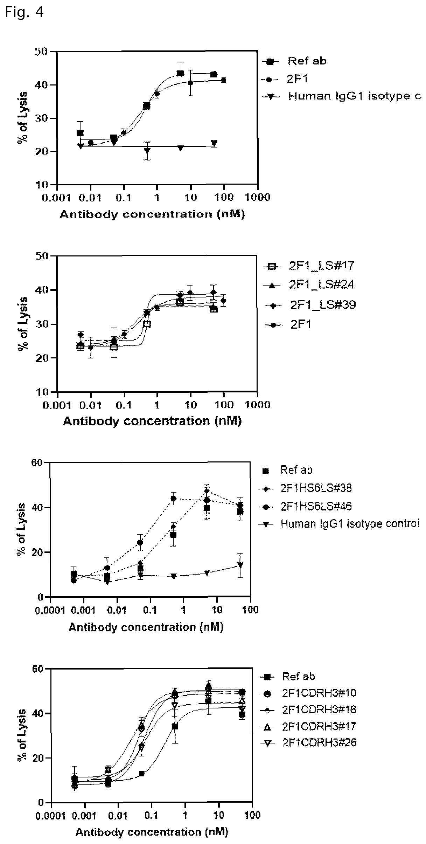

As a result, as shown in FIG. 4, the cancer cell killing activity of 2F1 and 2F1_LS#17 was comparable to that of the positive control antibody. In addition, 2F1HS6LS#38, 2F1HS6LS#46, 2F1CDRH3#10, 2F1CDRH3#16, 2F1CDRH3#17, and 2F1CDRH3#26 exhibited superior efficacy with approximately 2- to 10-fold increased cancer cell killing activity compared to the positive control antibody.

### Example 4.6. Analysis of enhanced cancer cell killing by natural killer cells across various indications

To determine whether the selected anti-HLA-G antibody 2F1CDRH3#10 binds to HLA-G expressed in various cancer types and inhibits its binding to ILT-2 expressed on natural killer cells (NK-92MI), thereby enhancing cancer cell killing by natural killer cells, the following analysis was performed. Cancer cell lines naturally expressing HLA-G, namely pancreatic cancer cells (HuP-T3), gastric cancer cells (SCH), bladder cancer cells, lung cancer cells, and triple-negative breast cancer cells, were dispensed into a 96-well plate at 1×10⁴ cells/well. Anti-HLA-G antibody was added at 10 nM, followed by incubation at 37°C under 5% CO₂ for 1 hour. NK-92MI cells were dispensed into wells containing bladder cancer cells and pancreatic cancer cells at 2×10⁴ cells/well, into wells containing gastric cancer cells and lung cancer cells at 5×10⁴ cells/well, and into wells containing triple-negative breast cancer cells at 5×10³ cells/well, followed by incubation at 37°C under 5% CO₂ for 24 hours. Thereafter, the procedure for determining the proportion of dead cells was carried out as described in Example 4.5.

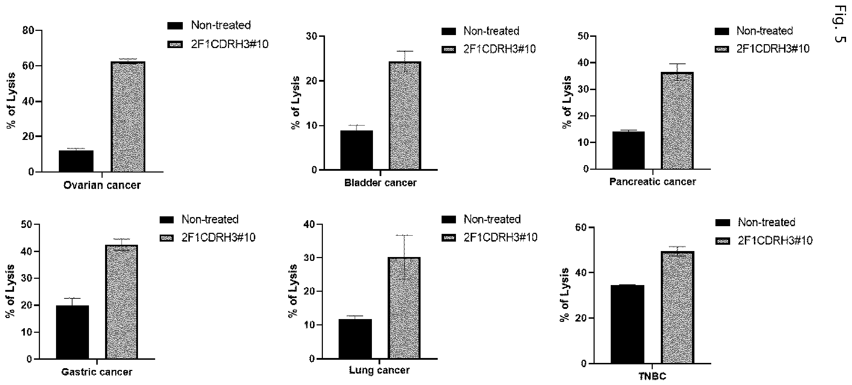

As a result, as shown in FIG. 5, the above results confirmed that 2F1CDRH3#10 induces natural killer cell-mediated cytotoxicity across various cancer types.

### Example 4.7. Analysis of antibody-dependent cellular phagocytosis by macrophages

To determine whether the selected anti-HLA-G antibody 2F1CDRH3#10 binds to HLA-G expressed on HLA-G-overexpressing cells and induces phagocytic activity of macrophages, the following analysis was performed. Macrophage cells (THP-1) and target cells (HLA-G-overexpressing cells) were stained using CellTrace Violet cell proliferation (Thermo Fisher Scientific) and CellTrace CFSE cell proliferation (Thermo Fisher Scientific) fluorescent staining kits, respectively, and then mixed by adding 1×10⁴ cells/well into a 96-well plate. 2F1CDRH3#10 and a human IgG1 isotype control antibody were serially diluted ten-fold from 10 nM, added to the 96-well plate containing co-cultured macrophages and target cells, and incubated at 37°C under 5% CO₂ for 24 hours. TrypLE^{™} Express (Gibco) was added, and the cells in each well were transferred to FACS tubes, followed by washing with FACS buffer (1,000 rpm, 3 minutes). Using FACSLyric^{™}, the proportion of macrophages positive for both CellTrace Violet and CFSE was determined.

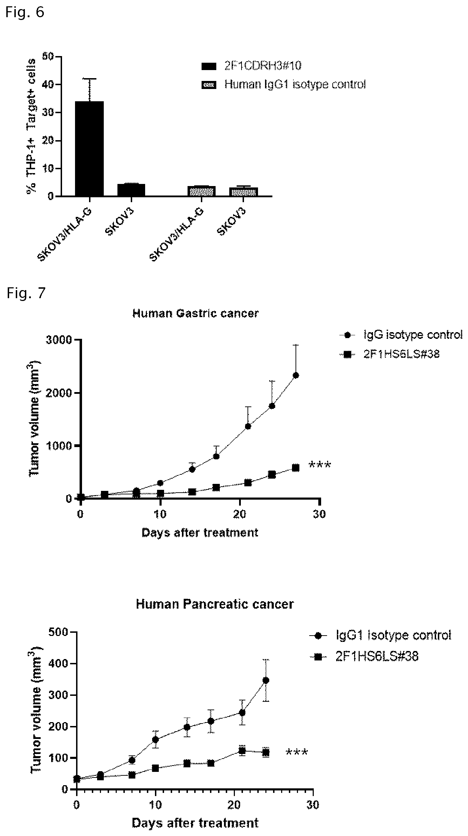

As a result, as shown in FIG. 6, it was confirmed that 2F1CDRH3#10 exhibits antibody-dependent cellular phagocytic activity.

### Example 5. Evaluation of in vivo biological activity of anti-HLA-G antibodies

BALB/c nude mice (6 weeks old) were injected with SCH gastric cancer cells and HuP-T3 pancreatic cancer cells, respectively, to establish in vivo xenograft mouse models. When the tumor volume was 100 mm³ or less, the antibody was administered via intravenous injection twice per week at a dose of 30 mg/kg per administration. Tumor volume changes following antibody administration were measured and are shown in Table 7 below.

As a result, as shown in Table 7 and FIG. 7, 2F1HS6LS#38 exhibited tumor growth inhibition in both mouse models and showed a statistically significant difference compared to the negative control.

**[Table 7]**

| Mouse model | Tumor growth inhibition (TGI, %) |
|---|---|
| Pancreatic cancer cells | 75.38 |
| Gastric cancer cells | 75 |

### Example 6. Evaluation of antibody-dependent cellular cytotoxicity of afucosylated antibodies

To induce afucosylation of antibodies, 2-FF (2-deoxy-2-fluoro-L-fucose), a fucose analog, was added to the culture medium at a concentration of 400 µM during production of the antibodies selected in Example 1.4, followed by purification, and ADCC activity was evaluated. Antibody-dependent cellular cytotoxicity (ADCC) was assessed in the same manner as described in Example 5.3.

As a result, as shown in FIG. 8 and Table 8 below, afucosylation increased the ADCC activity of the anti-HLA-G antibodies by approximately 6- to 15-fold. In addition, the afucosylated antibodies (six types) prepared from the antibodies selected in Example 1.4 exhibited ADCC activity approximately 2- to 10-fold higher than that of the afucosylated positive control antibody.

**[Table 8]**

| EC₅₀ (nM) | 2F1CDRH 3 #10 | 2F1CDRH 3 #16 | 2F1CDRH 3 #17 | 2F1CDRH 3 #26 | 2F1HS6 LS#38 | 2F1HS6 LS#46 | Positive control antibody (Ref ab) |
|---|---|---|---|---|---|---|---|
| IgG | 0.08 | 0.17 | 0.07 | 0.10 | 0.93 | 0.15 | 1.59 |
| Afucosyla tion | 0.01 | 0.02 | 0.01 | 0.02 | 0.07 | 0.02 | 0.13 |

### Example 7. Analysis of inhibitory activity of 2F1.eP against HLA-G and ILT-2 binding

To compare the inhibitory activity of 2F1 and 2F1.eP against binding between HLA-G and ILT-2, the inhibitory activity was analyzed using recombinant HLA-G antigen and the JEG-3 cell line. The inhibitory activity against binding between recombinant HLA-G antigen and ILT-2 was analyzed in the same manner as described in Example 5.1, and the inhibitory activity against binding between HLA-G and ILT-2 using the JEG-3 cell line was evaluated by FACS. JEG-3 cells (2×10⁵ cells/tube in 50 µL) were suspended in stain buffer and blocked on ice for 30 minutes. Recombinant human ILT-2 protein with a mouse Fc tag was diluted to a 4× concentration of 1.5 µM and added at 25 µL, followed by pre-incubation on ice for 30 minutes. Thereafter, 2F1 and 2F1.eP were added at 25 µL in stain buffer and incubated on ice for 1 hour. After adding 1 mL of stain buffer, the cells were washed three times by centrifugation (1,000 rpm, 3 minutes). The cell pellet was resuspended in 100 µL of stain buffer, and a secondary antibody, goat anti-mouse IgG Fc cross-adsorbed PE, was added at a dilution of 1:100 and incubated on ice for 45 minutes. After washing twice with 1 mL of stain buffer, analysis was performed using BD FACSLyric^{™}.

As a result, as shown in FIG. 9, it was confirmed that not only 2F1 but also 2F1.eP exhibited inhibitory activity against ILT-2 binding at a significant level.

### Example 8. Analysis of HLA subtype selectivity of anti-HLA-G antibodies

Binding of the antibodies obtained in Example 2 to HLA subtype antigens (97 different antigens including alleles of HLA-A, HLA-B, and HLA-C) was evaluated using a kit (LABScreen Single Antigen HLA Class I Combi, One Lambda). Antibodies at 1,000 nM were added to beads and incubated for 45 minutes in the dark. The beads were then washed twice with 1× wash buffer by centrifugation (1,300 × g, room temperature, 5 minutes). Anti-human IgG PE was added to each sample and incubated in the dark at room temperature for 30 minutes, followed by washing with 1× wash buffer. Analysis was performed using Luminex.

As a result, as shown in FIG. 10, all antibodies obtained in Example 2 exhibited MFI values of 1,000 or less, which is the cutoff value of the kit (LABScreen Single Antigen HLA Class I Combi), confirming selectivity for HLA-G.

### Example 9. Evaluation of in vitro cytotoxicity of ADC

The inhibitory effect of the antibody-drug conjugates on cell proliferation was evaluated in a pancreatic cancer cell line expressing HLA-G. Cells were seeded in a 96-well plate at 1×10³ cells/well. After 24 hours, the antibody-drug conjugates were added to the cancer cells at concentrations ranging from 0.256 pM to 100 nM (five-fold serial dilution), followed by incubation for 144 hours. Cell viability was measured using a CellTiter-Glo (CTG, Promega) assay. As a result, the CC₅₀ of the antibody-drug conjugate 2F1CDRH3#26-MMAE in the pancreatic cancer cell line was determined to be 0.83 nM.

## Claims

1. An antibody or an antigen-binding fragment thereof that specifically binds to human leukocyte antigen G (HLA-G).

2. The antibody or the antigen-binding fragment thereof according to claim 1, wherein the antibody or the antigen-binding fragment thereof comprises:
a heavy chain CDR1 having an amino acid sequence represented by SEQ ID NO: 1, 17, 33, 49, 64, 79, 94, 109, 125, 140, 156, 172, or 188;
a heavy chain CDR2 having an amino acid sequence represented by SEQ ID NO: 2, 18, 34, 50, 65, 80, 95, 110, 126, 141, 157, 173, or 189; and
a heavy chain CDR3 having an amino acid sequence represented by SEQ ID NO: 3, 19, 35, 51, 66, 81, 96, 111, 127, 142, 158, 174, or 190.

3. The antibody or the antigen-binding fragment thereof according to claim 1, wherein the antibody or the antigen-binding fragment thereof comprises:
a light chain CDR1 having an amino acid sequence represented by SEQ ID NO: 4, 20, 36, 52, 67, 82, 97, 112, 128, 143, 159, 175, or 191;
a light chain CDR2 having an amino acid sequence represented by SEQ ID NO: 5, 21, 37, 53, 68, 83, 98, 113, 129, 144, 160, 176, or 192; and
a light chain CDR3 having an amino acid sequence represented by SEQ ID NO: 6, 22, 38, 54, 69, 84, 99, 114, 130, 145, 161, 177, or 193.

4. The antibody or the antigen-binding fragment thereof according to claim 1, wherein the antibody or the antigen-binding fragment thereof comprises a heavy chain variable region having an amino acid sequence represented by SEQ ID NO: 15, 31, 47, 123, 154, 170, 186, or 202.

5. The antibody or the antigen-binding fragment thereof according to claim 1, wherein the antibody or the antigen-binding fragment thereof comprises a light chain variable region having an amino acid sequence represented by SEQ ID NO: 16, 32, 48, 63, 78, 93, 108, 124, 139, 155, 171, 187, or 203.

6. A nucleic acid encoding the antibody or the antigen-binding fragment thereof according to claim 1.

7. A vector comprising the nucleic acid according to claim 6.

8. An isolated host cell transformed with the vector according to claim 7.

9. A method for producing the antibody or the antigen-binding fragment thereof according to claim 1, comprising introducing the vector according to claim 7 into a host cell.

10. An antibody-drug conjugate (ADC) comprising the antibody or the antigen-binding fragment thereof according to claim 1.

11. A fusion protein comprising the antibody or the antigen-binding fragment thereof according to claim 1.

12. A pharmaceutical composition for preventing or treating cancer, comprising the antibody or the antigen-binding fragment thereof according to claim 1.

13. A composition for diagnosing cancer, comprising the antibody or the antigen-binding fragment thereof according to claim 1.

14. A kit for diagnosing cancer, comprising the antibody or the antigen-binding fragment thereof according to claim 1.

15. A method for providing information for diagnosing cancer, comprising:
detecting an HLA-G protein through an antigen-antibody reaction or measuring an expression level or activity of the HLA-G protein in a biological sample isolated from a subject suspected of having cancer using the antibody or the antigen-binding fragment thereof according to claim 1.

16. A method for providing information for treating cancer, comprising:
detecting an HLA-G protein through an antigen-antibody reaction or measuring an expression level or activity of the HLA-G protein in a biological sample isolated from a subject suspected of having cancer using the antibody or the antigen-binding fragment thereof according to claim 1.

17. A method for screening an anticancer agent, comprising:
(a) treating cancer cells with a candidate anticancer agent;
(b) measuring an expression level or activity of an HLA-G protein in the cancer cells treated with the candidate anticancer agent using the antibody or the antigen-binding fragment thereof according to claim 1; and
(c) determining the candidate anticancer agent treated in step (a) to be an anticancer agent when the expression level or activity of the HLA-G protein in step (b) is reduced compared to that of cancer cells not treated with the candidate anticancer agent.
